# EUROPÄISCHE PATENTSCHRIFT

(11) **EP 3 383 620 B1**
(45) Veröffentlichungstag und Bekanntmachung des Hinweises auf die Patenterteilung: **28.04.2021**
(21) Anmeldenummer: 16804752.0
(22) Anmeldetag: 29.11.2016
(51) Int. Cl.: B29C 65/20, B29C 65/74, B29C 65/30, A61M 39/14, B29C 65/78

(54) **VORRICHTUNG ZUM SCHWEISSEN VON SCHLÄUCHEN**
DEVICE FOR WELDING TUBES
DISPOSITIF DE SOUDAGE DE TUBES

(30) Priorität: 30.11.2015 CH 17512015
(43) Veröffentlichungstag der Anmeldung: 10.10.2018
(73) Patentinhaber: Reed Electronics AG, 6105 Schachen (CH)
(72) Erfinder: BÜHLER, Peter, 6103 Schwarzenberg (CH); FLUDER, Willy, 6102 Malters (CH); WYSS, Markus, 6103 Schwarzenberg (CH)
(74) Vertreter: Rentsch Partner AG
(86) Internationale Anmeldenummer: PCT/EP2016/079073
(87) Internationale Veröffentlichungsnummer: WO 2017/093216

(56) Entgegenhaltungen:
- EP-A1- 0 643 975
- WO-A2-2008/005882
- CH-B1- 698 798

## Beschreibung

### TECHNISCHES GEBIET

Die Erfindung betrifft eine Vorrichtung zum Verschweissen von thermoplastischen Schläuchen und, ein Verfahren zum Verschweissen sowie ein nicht als solches beanspruchtes Schweissmesser.

### STAND DER TECHNIK

Aus der US4610670 A ist eine Vorrichtung und ein Verfahren zur sterilen Verbindung von zwei thermoplastischen Schläuchen bekannt. Bei diesem Verfahren werden die Enden zweier Schläuche miteinander verbunden, die jeweils mit einem Ende in einen Blutbeutel münden. Die beiden Schläuche werden hierzu parallel zu einander in zwei von einander beabstandete Blöcke eingelegt, mit einem beheizten Messer durchtrennt und zu einem durchgängigen Schlauch gefügt. Ziel ist es, eine sterile Verbindung zwischen zwei Schläuchen zu schaffen.

Die EP0208004 A1 offenbart ein Verfahren und eine Vorrichtung zum sterilen Docken von Kunststoffschlauchabschnitten. Vergleichbar mit der US461 0670 A werden auch hier zwei Schlauchenden zu einem durchgängigen Schlauch gefügt mit dem Unterschied, dass zwei bereits geschnittene Schlauchenden in Halteblöcke eingespannt, durch eine Heizeinrichtung erwärmt und durch eine horizontale Verschiebung der Halteblöcke zueinander gefügt und miteinander verschweisst werden.

Die US 2012/0269679 A1 offenbart ein System mit welchem eine Vielzahl von Schläuchen, welche an ihrem einen Ende je mit einem Blutbeutel verbunden sind mit einer Vielzahl von Schläuchen, welche in einem sogenannten "Pooling-Container" münden, verschweisst werden. Im offenbarten Verfahren werden einzelne beheizte Klingen eingesetzt, um die Schläuche zu trennen und in einem weiteren Schritt miteinander zu verschweissen.

Die CH698798 B1 beschreibt ein Verfahren und Vorrichtung zum Verbinden zweier Leitungen aus thermoplastischem Kunststoff durch Verschweissen, wobei die zu verschweissenden Leitungen einander überlappend in zwei relativ und parallel zueinander verfahrbaren Halterungen angeordnet und mittels einer beheizten Trennklinge unter Aufschmelzen des Kunststoffes gemeinsam durchtrennt werden. Daraufhin werden die Halterungen parallel soweit gegeneinander verfahren, bis die Trennstellen der zu verbindenden Leitungen einander gegenüberstehen. Um das Austreten von Flüssigkeiten aus den resultierenden Restende zu verhindern, ist an jeder Halterung auf jeder Seite ein geschlossener Hilfsleitungsteil vorgesehen. Die Restenden werden jeweils mit einem geschlossenen Hilfsleitungsteil verbunden.

Die EP0643975 A1 beschreibt ein Zellspeicherbeutelsystem mit einem Aufbewahrungsbeutel zum Aufnehmen von Zellen, welcher mittels eines Verbindungsrohrs aufweisend ein geschlossenes Ende verbunden ist. Ferner umfasst das System einen Vorratsbehälter zum Aufnehmen eines weiteren Fluids und einen Abfallfluidbehälter zum Aufnehmen von Abfallfluid. In dem System ist das mindestens eine Verbindungsrohr geeignet, mit einem Fluidzufuhrschlauch und/oder dem Abfallfluidschlauch mittels einer Rohrverbindungsvorrichtung steril verbunden zu werden.

### DARSTELLUNG DER ERFINDUNG

Bei Verarbeitung von Blutkonserven kommen hoch automatisierte Prozessabläufe zum Einsatz. An die hierfür eingesetzte Maschine werden daher hohe Anforderungen bezüglich Effizienz und Sterilität gestellt. Ein wesentlicher Schritt ist das Verbinden mehrerer Blutbeutel miteinander, in dem die einzelnen Schläuche, welche von den einzelnen Beuteln wegführen, miteinander verschweisst werden. In Blutbanken werden zur Verbindung der einzelnen Blutkonserven pro Tag über 2000 Schweissungen an hunderten von Blutbeuteln vorgenommen, um beispielsweise aus Spenderblut Thrombozytenkonzentrat herzustellen. Es ist daher notwendig, möglichst viele Schweissungen innerhalb möglichst kurzer Zeit und von hoher und gleich bleibender Qualität durchführen zu können. Es soll eine sterile Verbindung hergestellt werden. Aufgabe der Erfindung ist es, eine Vorrichtung und ein Verfahren zum Verschweissen einer Vielzahl von thermoplastischen Schläuchen zur Verfügung zu stellen, welche die vorgängig erwähnten Vorteile aufweist und Nachteile des Standes der Technik vermeidet.

Eine weitere Aufgabe ist es, eine Vorrichtung, ein Verfahren und ein als solches nicht beanspruchtes beheizbares Schweissmesser zur Verfügung zu stellen, wobei eine Vielzahl von thermoplastischen Schläuchen geschnitten und deren Enden mit gleich bleibender Qualität verschweisst werden sollen. Die Aufgaben werden durch die Anspruchsmerkmale gelöst.

Bevorzugte Ausführungsarten der Erfindung sind in den abhängigen Ansprüchen angegeben.

Die erfindungsgemässe Vorrichtung zum Verschweissen von thermoplastischen Schläuchen einer ersten und zweiten Schlauchgruppe umfasst zwei Schlauchhalter mit je einer ersten und einer zweiten Schlauchklemme, sowie ein Schweissmesser. Die Schläuche der ersten Schlauchgruppe verlaufen gerade durchlaufend zwischen den beiden Schlauchhaltern, werden in die Durchgangsöffnungen der ersten beiden Schlauchklemmen eingelegt und darin gequetscht.

Die Schläuche der zweiten Schlauchgruppe verlaufen ebenfalls gerade durchlaufend zwischen den beiden Schlauchhaltern, werden in Durchgangsöffnungen der beiden zweiten Schlauchklemmen eingelegt und darin gequetscht.

Das Schweissmesser wird zwischen dem ersten und dem zweiten Schlauchhalter bewegt und ist dazu eingerichtet, die gequetschten Schläuche der ersten und der zweiten Schlauchgruppe in einer Bewegung zu durchtrennen, um auf diese Weise Schnitt-Enden und Rest-Enden zu erzeugen.

Einer der beiden Schlauchhalter kann relativ zum anderen Schlauchhalter verschoben werden, um dadurch die Schnitt-Enden der Schläuche aufeinander auszurichten.

Die beiden Schlauchhalter können ferner in einer horizontalen Richtung zu einander oder weg von einander verschoben werden, um dadurch je ein Schnitt-Ende eines Schlauchs der ersten Schlauchgruppe mit je einem Schnitt-Ende eines Schlauchs der zweiten Schlauchgruppe zu je einem durchgängigen Schlauch gleichzeitig zu verschweissen.

Ein Vorteil der erfindungsgemässen Vorrichtung ist, dass auf diese Weise mit einer geringen Anzahl an Arbeitsschritten eine Vielzahl von thermoplastischen Schläuchen steril zu durchgängigen Schläuchen verschweisst werden können.

Erfindungsgemäss sind die Durchgangsöffnungen der beiden ersten Schlauchklemmen auf einer ersten Ebene und die Durchgangsöffnungen der beiden zweiten Schlauchklemmen auf einer zweiten Ebene angeordnet, wobei die erste Ebene parallel und beabstandet zur zweiten Ebene angeordnet ist.

In einer bevorzugten Ausführungsform der Erfindung, weisen die beiden ersten Schlauchklemmen und die beiden zweiten Schlauchklemmen Quetschzonen auf, wobei diese Quetschzonen entlang der Durchgangsöffnungen verlaufen und dazu eingerichtet sind, die eingelegten Schläuche in einem schrägen Winkel zur ersten bzw. zur zweiten Ebene zu quetschen.

Vorzugsweise ist das Schweissmesser in einem Schweissmesserhalter einklemmbar und eine Antriebseinheit ist eingerichtet, den Schweissmesserhalter zum Durchtrennen der Schläuche in einer vertikalen Richtung zu bewegen. Bei dieser Bewegung in vertikaler Richtung wird einer der beiden Schlauchhalter relativ zum anderen Schlauchhalter zum Ausrichten der Schnitt-Enden der Schläuche vertikal verschoben. Des Weiteren werden erfindungsgemäss durch die Bewegung des Schweissmesserhalters in vertikale Richtung die beiden Schlauchhalter in der horizontalen Richtung aufeinander zu oder voneinander weg oder beide Schlauchhalter gleichzeitig in eine Richtung bewegt.

In einer bevorzugten Ausführungsform weist die Vorrichtung eine in der vertikalen Richtung bewegbare Plattform auf und der Schweissmesserhalter ist auf dieser Plattform angeordnet.

Vorzugsweise weist der Schweissmesserhalter zwei sich gegenüberliegende Klemmteile auf, zwischen welche das Schweissmesser einklemmbar ist. Vorzugsweise ist eines der beiden Klemmteile feststehend und das andere relativ beweglich zum feststehenden Klemmteil angeordnet.

Ferner ist an einem der beiden Klemmteile ein Paar von ersten Kulissenträgern vorgesehen. In einer Ausführungsform ist das Klemmteil und das Paar von ersten Kulissenträgern als ein Stück gefertigt. Alternativ ist das Paar von ersten Kulissenträgern an einem der beiden Klemmteile als separate Teile montiert.

Das Paar von Kulissenträgern weist vorzugsweise insgesamt vier erste Kulissen auf. Diese Kulissen sind untereinander identisch und spiegelsymmetrisch zu einer Ebene des Schweissmesser angeordnet, wenn das Schweissmesser zwischen die beiden Klemmteile eingeklemmt ist.

Ferner sind Abtasträderpaare vorgesehen, die bei der Bewegung des Schweissmesserhalters in vertikaler Richtung die Kulissen abtasten und dabei die Schlauchhalter in der horizontalen Richtung zueinander oder weg voneinander oder beide Schlauchhalter gleichzeitig in eine Richtung bewegen.

Vorzugsweise ist ein zweiter Kulissenträger und ein weiteres Abtastrad vorgesehen. Das weitere Abtastrad ist dazu eingerichtet, bei der Bewegung des Schweissmesserhalters in vertikaler Richtung den zweiten Kulissenträger abzutasten. Dabei wird das eine Klemmteil relativ zum zweiten feststehenden Klemmteil bewegt und auf diese Weise der Abstand zwischen den beiden Klemmteilen variiert.

Beim Abtasten des zweiten Kulissenträgers durch das Abtastrad wird der Abstand zwischen den beiden Klemmteilen vorzugsweise in drei unterschiedlichen Positionen variiert. In einer Aufnahmeposition ist das Abtastrad in Kontakt mit dem zweiten Kulissenträger und es resultiert ein Abstand zwischen den beiden Klemmteilen, welcher gerade für das Einschieben des Schweissmessers benötigt wird.

In einer Kontaktierungsposition ist der Abstand zwischen den beiden Klemmteilen so gewählt, dass das Schweissmesser eingeklemmt und gleichzeitig elek-trisch kontaktiert ist. In dieser Position ist das Abtastrad nicht in Kontakt mit dem zweiten Kulissenträger.

In einer Freigabeposition ist das Abtastrad in Kontakt mit dem zweiten Kulissenträger, und der Abstand zwischen den beiden Klemmteilen ist so gewählt, dass das Schweissmesser ausgeworfen werden kann.

In einer bevorzugten Ausführungsform sind an mindestens einem der beiden Klemmteile Druckstücke angeordnet, welche das Schweissmesser in der Kontaktierungsposition kontaktieren. In einer Ausführungsform sind diese Druckstücke an dem mindestens einen Klemmteil federnd gelagert. Durch die federnde Lagerung kann der Druck welcher bei der Kontaktierung auf das Schweissmesser ausgeübt wird, variabel eingestellt werden. Es hat sich herausgestellt, dass eine möglichst regelmässige Druckverteilung auf das Schweissmesser eine regelmässige Temperaturverteilung über die Fläche des Schweissmessers bewirkt. Eine möglichst gleichmässige Verteilung ist wünschenswert, da dies die Qualität der Verschweissung verbessert. Es ist dann sichergestellt, dass auf die Schnitt-Enden bzw. die Rest-Enden der thermoplastischen Schläuche eine vergleichbare Wärmeenergie, unabhängig von deren Lage im Schlauchhalter, aufgebracht wird.

Erfindungsgemäss weist die Vorrichtung eine Transformatoreinheit mit einer Primärwicklung und einer Sekundärwicklung auf, wobei die Sekundärwicklung durch einen einzigen Leiter gebildet wird. Dieser Leiter ist mit einem der beiden Klemmteile, vorzugsweise mit dem feststehenden Klemmteil, verbunden. Das Schweissmesser schliesst in der Kontaktierungsposition den sekundären Stromkreis und bildet darin das Haupt-Widerstandelement, in dem die Verlustleistung überwiegend anfällt. Das Schweissmesser wird so auf die gewünschte Temperatur aufgeheizt. Das Schweissmesser wird mit einem hohen Wechselstrom auf eine Temperatur von rund 300 °C aufgeheizt. Mit der erfindungsgemässen Anordnung konnte eine konstante Heizleistung am Schweissmesser erzielt werden.. Durch den erfindungsgemässen Aufbau kann die Elektrik gut in das Gehäuse der Vorrichtung integriert werden. Erfindungsgemäss weist die erste und die zweite Schlauchgruppe je die gleiche Anzahl an einzelnen Schläuchen, vorzugsweise mehr als zwei, insbesondere sechs Schläuche pro Schlauchgruppe, auf.

Das erfindungsgemässe Verfahren zum Verschweissen einer Vielzahl von Schläuchen einer ersten und einer zweiten Schlauchgruppe mit der erfindungsgemässen Vorrichtung umfasst mehrere Schritte.

Zu Beginn werden die Schläuche der ersten und der zweiten Schlauchgruppe parallel und übereinander in sich gegenüberliegende erste und zweite Schlauchhalter gerade durchlaufend eingelegt und gequetscht. Ein beheizbares Schweissmesser wird in einen Schweissmesserhalter eingelegt und auf eine gewünschte Temperatur erhitzt.

Das beheizte Schweissmesser wird zwischen dem ersten und dem zweiten Schlauchhalter bewegt, wobei die gequetschten Schläuche gleichzeitig in Schnitt-Enden und Rest-Enden durchtrennt werden.

Die Schnitt-Enden der Schläuche werden durch das beheizte Schweissmesser aufgeschmolzen.

Die Schnitt-Enden der Schläuche der ersten Schlauchgruppe werden auf die Schnitt-Enden der Schläuche der zweiten Schlauchgruppe ausgerichtet, in dem einer der beiden Schlauchhalter relativ zum anderen Schlauchhalter verschoben wird, sodass die Schnitt-Enden der Schläuche der ersten Schlauchgruppe symmetrisch gegenüber liegend zu den Schnitt-Enden der zweiten Schlauchgruppe angeordnet sind und das Schweissmesser herausgezogen werden kann.

Die beiden Schlauchhalter werden aufeinander zu verschoben, um dadurch je ein Schnitt-Ende der Schläuche der ersten Schlauchgruppe mit je einem Schnitt-Ende der Schläuche der zweiten Schlauchgruppe zu je einem durchgängigen Schlauch gleichzeitig zu verschweissen.

Zum Schluss wird das Schweissmesser aus dem Schweissmesserhalter ausgeworfen.

Beim Einlegen des Schweissmessers (4) ist der horizontale Abstand zwischen dem ersten und dem zweiten Schlauchhalter (100, 200) d1. Beim Durchtrennen der gequetschten Schläuche wird der Abstand auf d4 vergrösserst . Beim Aufschmelzen der Schnitt-Enden beträgt der Abstand d2, wobei gilt d1<d2<d4. Beim Verschieben einer der beiden Schlauchhalter (100, 200) wird der Abstand wieder auf d4 vergrössert. Beim Verschweissen ist der Abstand d3, wobei gilt d3 <d4 und beim Auswerfen des Schweissmessers beträgt der Abstand zwischen den beiden Schlauchhaltern (100, 200) d5, wobei d5 den kleinsten aller fünf Abstände darstellt.

Der Abstand d4 ist in seiner Grösse so gewählt, dass an den thermoplastischen Schläuchen eine Zugspannung anliegt. Ein Durchtrennen wird dadurch erleichtert. Beim Aufschmelzen der Schnitt-Enden wird der Abstand auf d2 verringert. Auf diese Weise berühren die Schnitt-Enden und die Rest-Enden das beheizte Schweissmesser und werden an Ihren Querschnitten entsprechend aufgeschmolzen. Beim Verschieben einer der beiden Schlauchhalter wird der Abstand wieder auf d4 vergrössert. Durch die Vergrösserung des Abstandes auf d4 ist sichergestellt, dass beim Herausziehen des Schweissmessers ausreichend Schmelzmaterial auf den Schlauchquerschnitten verbleibt, welches dann für die nachfolgende Verschweissung zur Verfügung steht. Danach werden je ein Schnitt-Ende eines Schlauchs der ersten Schlauchgruppe mit je einem Schnitt-Ende eines Schlauchs der zweiten Schlauchgruppe zu je einem durchgängigen Schlauch gleichzeitig verschweisst, in dem die Enden durch eine Verringerung des Abstandes zwischen den Schlauchhaltern auf d3 gefügt werden. Zum Schluss wird das Schweissmesser ausgeworfen und die verschweissten Schläuche entnommen. Die Schlauchhalter befinden sich im Abstand d5 zueinander, der im Regelfall so gering ist, dass sie einander berühren.

Das als solches nicht beanspruchte Schweissmesser, welches bevorzugt in der erfindungsgemässen Vorrichtung bzw. beim erfindungsgemässen Verfahren zum Einsatz kommt, weist in seiner Schnittfläche eine Vielzahl von Ausnehmungen auf. Erfindungsgemäss dient die Schnittfläche dazu die Schnitt-Enden bzw. die Rest-Enden der Schläuche aufzuschmelzen, weshalb im Folgenden von einer Schmelzfläche die Rede ist.

Es konnte festgestellt werden, dass die Ausnehmungen und insbesondere deren Anordnung und/oder Anzahl einen Einfluss auf die vertikale sowie horizontale Temperaturverteilung bezogen auf die Schweissmesserfläche hat. Eine regelmässige Verteilung ist vorteilhaft, da auf diese Weise ein möglichst gleichmässiges Aufschmelzen der Schnitt-

Enden der thermoplastischen Schläuche sichergestellt ist und ein gutes Schweissergebnis sichergestellt wird.

In einer bevorzugten Ausführungsform weist die Schmelzfläche eine rechteckige Form auf und entlang der beiden kurzen Seiten des Rechtecks sind Randzonen vorgesehen, welche dazu eingerichtet sind, eine Sekundärseite einer Transformatoreinheit zu kontaktieren. Vorzugsweise erfolgt diese Kontaktierung über die Druckstücke, welche an einem der beiden Klemmteile angeordnet sind.

Die Ausnehmungen in der Schmelzfläche sind entlang der Randzonen der Schmelzfläche angeordnet.

Das nicht als solches beanspruchte Schweissmesser weist an einer der beider längeren Seiten des Rechtecks eine Klinge auf, wobei die Klinge bezogen auf den Querschnitt des Rechtecks eine symmetrische Form aufweist. Diese Form ist vorteilhaft, da das Schweissmesser beim Durchtrennen der Schläuche dadurch einen sehr geraden Schnitt erzeugt.

In einer Ausführungsform wird das Schweissmesser durch hälftige Längsfaltung eines Blättchens von doppelter Breite hergestellt. Als Klinge, welche die Schläuche schneidet, dient die Biegekante entlang welcher die beiden Hälften des Blättchens zusammenhängen. Auf diese Weise resultiert eine symmetrische abgerundete Klinge.

Alternativ zur Faltung wird das Schweissmesser durch Stanzen und dessen Klinge durch Prägung hergestellt. Die Prägung wird entlang einer Längsseite des Blättchens vorgenommen, in dem die gewünschte Klingenform entlang der Längsseite eingepresst wird. Durch diese Gestaltung wird in rationeller Weise eine nicht zu scharfe, sondern abgerundete, vor allem jedoch gleichmässige und zu beiden Seiten hin symmetrische Schneidkante geschaffen.

Die jedoch als solches nicht beanspruchten Schweissmesser werden in einer Box bevorratet, die vorzugsweise als Ganzes austauschbar ist.

### KURZE ERLÄUTERUNG ZU DEN FIGUREN

Die Erfindung soll nachfolgend anhand eines Ausführungsbeispiels im Zusammenhang mit der Zeichnung näher erläutert werden. Es zeigen:
- Fig. 1: zeigt eine perspektivische Ansicht der erfindungsgemässen Vorrichtung in einer Ansicht von schräg oben mit Tisch und Tischbeinen,
- Fig. 1A: zeigt eine perspektivische Ansicht von oben auf die erfindungsgemässe Vorrichtung mit geschlossenen Schlauchhaltern und eingelegten Schläuchen,
- Fig. 2: zeigt eine Ansicht auf die erfindungsgemässe Vorrichtung von unten,
- Fig. 3: zeigt einen Teil der erfindungsgemässen Vorrichtung in einer perspektivischen Ansicht mit geöffneten Schlauchhaltern,
- Fig. 4: zeigt eine Schnittdarstellung der erfindungsgemässen Vorrichtung, wobei sich der Schweissmesserhalter in einer Position befindet, in welcher das Schweissmesser aufgenommen werden kann **("Bladefeed Position"),**
- Fig. 5: zeigt eine Schnittdarstellung der erfindungsgemässen Vorrichtung in einer Position, in welcher das Schweissmesser in die Schweissmesserhalterung eingelegt und elektrisch kontaktiert ist **("Precut Position"),**
- Fig. 6: zeigt eine Schnittdarstellung der erfindungsgemässen Vorrichtung in einer Position, in welcher der erste und der zweite Schlauchhalter derart beabstandet voneinander angeordnet sind, dass die eingelegten Schläuche gedehnt werden **("Stretch Position"),**
- Fig. 7: zeigt eine Schnittdarstellung der erfindungsgemässen Vorrichtung in einer Position, in welcher die Schläuche durch das Schweissmesser durchtrennt werden und der erste und zweite Schlauchhalter in vertikaler Richtung gegen einander verschoben und die Schläuche aufeinander ausgerichtet sind **("Cut Position"),**
- Fig. 8: zeigt eine weitere Schnittdarstellung der Ausführungsform mit eingelegten Schläuchen, wobei der erste und der zweite Schlauchhalter in vertikaler Richtung gegeneinander verschoben und die zu verschweissenden Schlauchenden aufeinander ausgerichtet und gefügt sind **("Press Position")** und das Schweissmesser sich nicht mehr zwischen den Schlauchhaltern befindet,
- Fig. 9: zeigt eine Schnittdarstellung der erfindungsgemässen Vorrichtung in einer Position, in welcher das Schweissmesser ausgeworfen wird **("Eject Position"),**
- Fig. 10: zeigt eine bevorzugte Ausführungsform eines als solches nicht beanspruchten Schweissmessers,
- Fig. 11: zeigt die vertikale und horizontale Temperaturverteilung am als solches nicht beanspruchten Schweissmesser entlang einer ersten, zweiten und dritten Linie.

In den verschiedenen Figuren sind dieselben Teile mit denselben Bezugszeichen versehen.

Von mehrfach vorhandenen, gleichartigen Teilen ist jeweils nur eines mit einem Bezugszeichen versehen.

### WEGE ZUR AUSFÜHRUNG DER ERFINDUNG

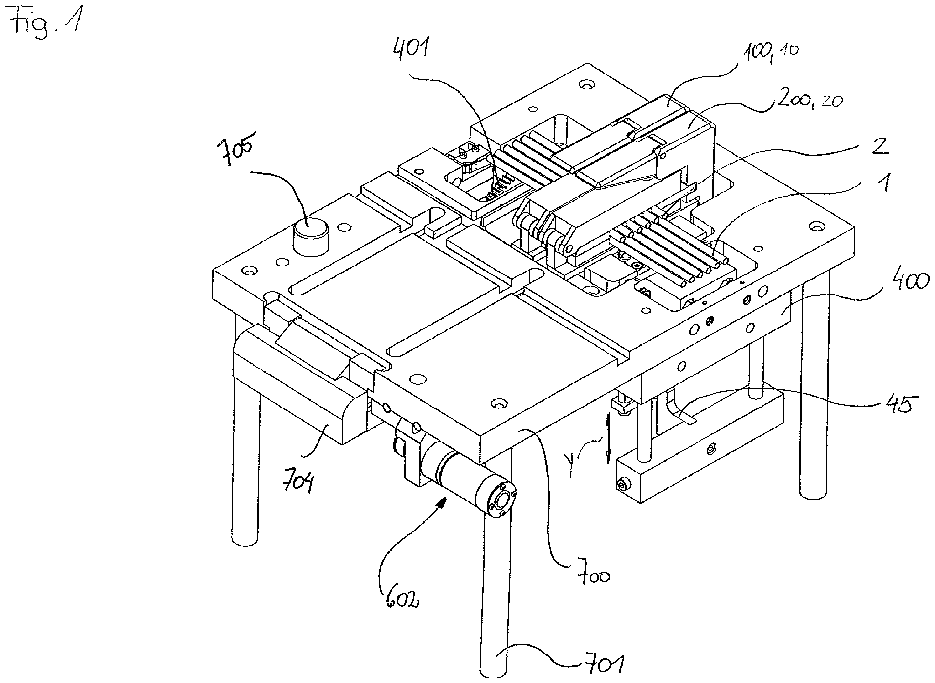

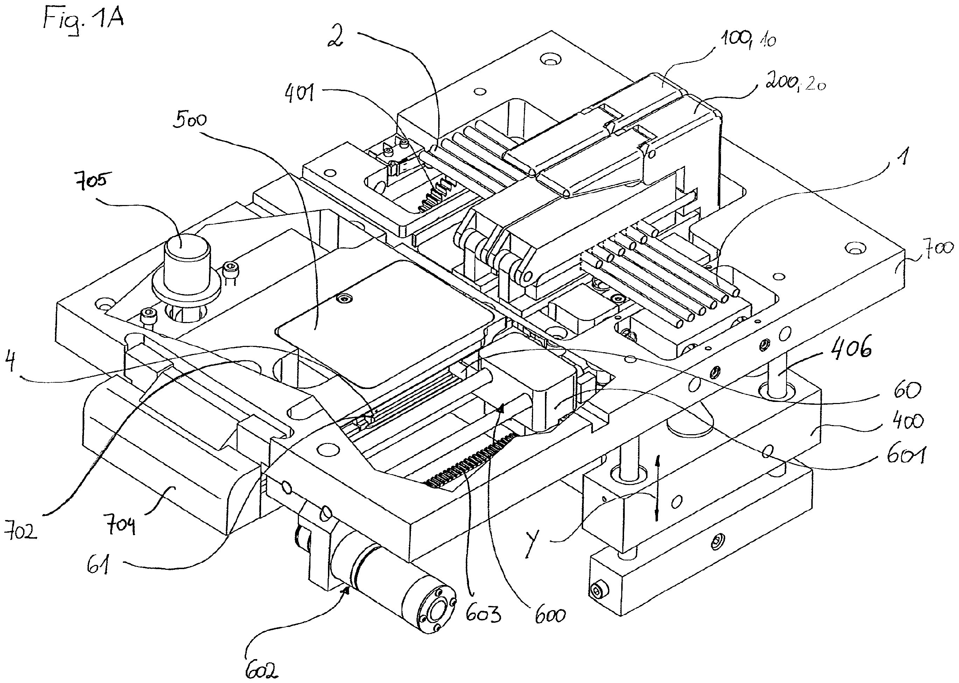

**Figur** 1 zeigt eine perspektivische Ansicht von oben auf die erfindungsgemässe Vorrichtung des Ausführungsbeispiels, die auf bzw. an einem Tisch 700 aufgebaut ist. Der Tisch steht auf vier Beinen 701. Die Vorrichtung weist zwei Schlauchhalter 100, 200 auf, in welche Schläuche eingelegt sind, wobei die Schläuche einer ersten Schlauchgruppe 1 auf einer ersten Ebene und die Schläuche einer zweiten Schlauchgruppe 2 auf einer zweiten Ebene darüber verlaufen. Die beiden Schlauchhalter 100, 200 weisen zusätzlich Spannhebel 10, 20 auf.

In Figur 1A ist durch einen zu diesem Zweck erzeugten Ausschnitt 702 des Tisches 700 eine Box 500 sichtbar, in welcher Schweissmesser bevorratet sind, und eine Schweissmesserzuführung 600 zu einem Schweissmesserhalter 40. Letzterer ist unterhalb der Schlauchhalter 100, 200 angeordnet, so dass er in beiden Figuren 1 und 1A nicht sichtbar ist. Die Schweissmesserzuführung 600 umfasst einen Transportschlitten 601 für einen Schieber 60 und einen Antrieb 602, bei dem es sich beispielsweise um einen "DC Planetary Gear Brush Motor" handelt. Der Schieber 60 ist mit dem Antrieb 602 über einen Zahnriemen 603 verbunden. Die Box 500 ist derart ausgestaltet, dass die einzelnen Schweissmesser 4 in Längsrichtung aufgestellt in der Box 500 einsortiert sind (in Figur 1 nicht sichtbar). Die Box 500 ist an jener Seite, welche zur Schweissmesserzuführung 600 hin orientiert ist, offen. Ein Federelement, angeordnet in der Box (in Figur 1 nicht sichtbar) drückt das Schweissmesser 4, welches für den nächsten Schweissvorgang vorgesehen ist, in die Führungsschienen 61 der Schweissmesserzuführung 600.

Unter dem Tisch 700 angeordnet ist eine Plattform 400, auf welcher der Schweissmesserhalter 40 (in Figur 1 nicht sichtbar) montiert ist. Die Plattform 400 und Schweissmesserhalter 40 kann mit Hilfe einer weiteren Antriebseinheit 401 auf den Tisch 700 zu bzw. von diesem weg bewegt werden (Pfeil Y), d. h. wenn der Tisch horizontal ausgerichtet ist, in vertikaler Richtung auf und ab.

In Figur 1 sowie in den weiteren Figuren ist auf die Darstellung eines üblicherweise zusätzlich vorhanden Gehäuses verzichtet. Aus dem Gehäuse ragen bevorzugt die Schlauchhalter 100, 200 nach oben heraus, so dass das Gehäuse zum Einlegen oder zum Herausnehmen der Schläuche nicht geöffnet werden muss. Ausserdem sollte die Box 500 mit dem Schneidmesservorrat für einen Austausch einfach zugänglich sein, was mit Hilfe einer Schublade 704 möglich gemacht wird. Bedienelemente wie ein Startknopf 705 können ebenfalls an dem Gehäuse vorgesehen sein (siehe beispielsweise Figur 1, 1A).

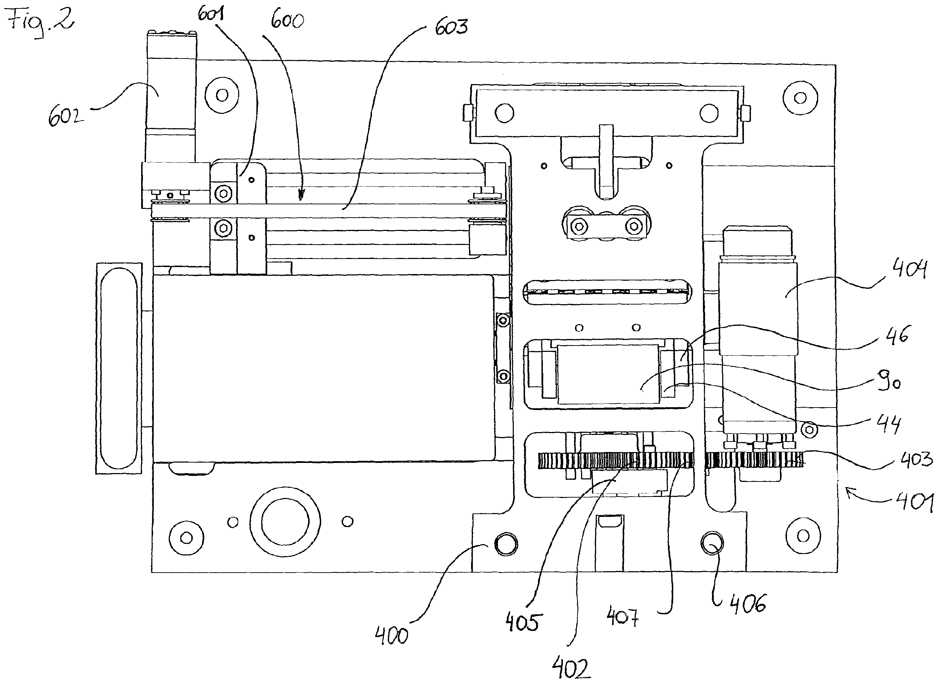

**Figur** 2 zeigt die erfindungsgemässe Vorrichtung in einer Ansicht von unten, wobei die Plattform 400, auf die der Schweissmesserhalter 40 aufgesetzt ist, und mehr von der Antriebseinheit 401 zu sehen ist, welche die Plattform 400 in einer vertikalen Richtung bewegt. Sie umfasst drei Zahnräder 402 403 und 407, wobei das Zahnrad 402 über ein Pleuel 405 mit der Plattform 400 verbunden ist. Das Zahnrad 407 befindet sich zwischen dem Zahnrad 402 und 403 (in Figur 2 sichtbar). Das Pleuel 405 setzt die kreisförmige Bewegung des Zahnrads 402 in eine lineare Auf- und Abbewegung der Plattform 400 entlang stangenförmiger Führungen 406 um. Das Zahnrad 403 wird durch einen Motor 404 angetrieben, bei dem es sich beispielsweise wieder um einen "DC Planetary Gear Brush Motor" handelt. In Fig. 2 sichtbar ist auch eine Transformatoreinheit 90 zur Heizung des Schweissmessers 4, die nachstehend noch näher beschrieben wird.

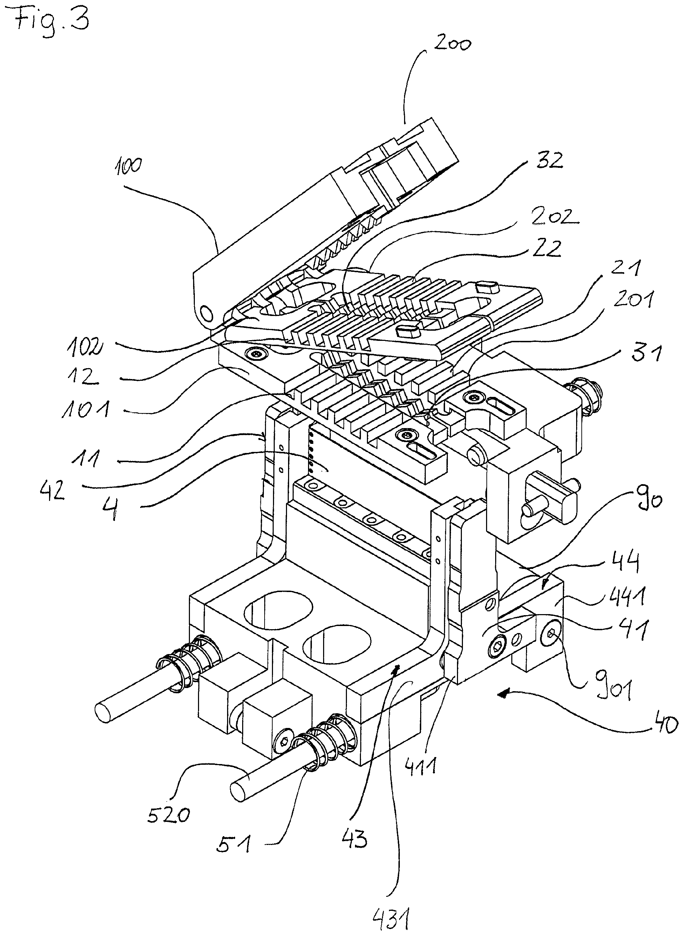

**Figur 3** zeigt einen Teil der erfindungsgemässen Vorrichtung in einer perspektivischen Ansicht mit geöffneten Schlauchhaltern 100, 200, ohne eingelegte Schläuche. Der erste und der zweite Schlauchhalter 100, 200 umfassen je eine erste 101, 201 und eine zweite Schlauchklemme 102, 202. Die erste Schlauchklemme 101 des ersten Schlauchhalters 100 und die erste Schlauchklemme 201 des zweiten Schlauchhalters 200 weisen je ein Unterteil und ein Oberteil auf. Die zweite Schlauchklemme 102 des ersten Schlauchhalters 100 und die zweite Schlauchklemme 202 des zweiten Schlauchhalters 200 weisen ebenfalls je ein Unterteil und ein Oberteil auf. Das Oberteil der ersten Schlauchklemme 101 des ersten Schlauchhalters 100 und das Oberteil der ersten Schlauchklemme 201 des zweiten Schlauchhalters 200 bilden gleichzeitig das Unterteil der zweiten Schlauchklemme 102, 202 des ersten bzw. zweiten Schlauchhalters 100, 200. Jede Schlauchklemme weist Durchgangsöffnungen 11, 12, 21 und 22 auf, in welche die Schläuche einer ersten und einer zweiten Schlauchgruppe eingelegt werden. Die Durchgangsöffnungen 11, 21 der beiden ersten Schlauchklemmen 101, 201 sind auf einer ersten Ebene und die Durchgangsöffnungen 12, 22 auf einer zweiten Ebene angeordnet. Die erste Ebene ist parallel und vertikal beabstandet zur zweiten Ebene. Die beiden ersten Schlauchklemmen 101, 201 sowie die beiden zweiten Schlauchklemmen 102, 202 weisen erste und zweite Quetschzonen 31, 32 auf, welche dazu eingerichtet sind, die eingelegten Schläuche vor dem Durchtrennen zu quetschen. Die Quetschzonen 31, 32 sind sägezahnförmig ausgebildet, so dass die Schläuche unter einem schrägen Winkel, insbesondere von 25°- 30° gequetscht werden. Durch die Quetschung werden die Schläuche komplett verschlossen, so dass insbesondere in den Schläuchen enthaltene Flüssigkeit beispielsweise von Blutkonserven die Quetschzonen nicht mehr passieren kann.

In der Figur 3 sichtbar ist weiter der Schweissmesserhalter 40 mit einem ersten Klemmteil 43, welches gegenüberliegend zu einem zweiten Klemmteil 44 angeordnet ist und relativ zu diesem zweiten, feststehenden Klemmteil entlang von stangenförmigen Führungen 520 beweglich ist. Die beiden Klemmteile weisen jeweils ein Paar von abgewinkelten Klemmarmen 431, 441 auf, wobei das Schweissmesser 4 zwischen den nach oben aufragenden Abschnitten dieser Klemmarme gehalten wird. Zum Anpressen des ersten Klemmteils 43 gegen das zweite Klemmteil 44 kommen erste Federn 51 zum Einsatz.

Am Klemmteil 44 befestigt ist ein Paar von Kulissenträgern 41, an deren aufragenden Abschnitten beidseitig und damit insgesamt vier erste Kulissen 411 ausgeformt sind. Alternativ ist das Klemmteil 44 und das Paar von Kulissenträgern 41 als ein Teil ausgebildet. Die Kulissen 411 sind untereinander identisch und spiegelsymmetrisch zur Ebene des Schweissmessers 4 angeordnet, wenn dieses zwischen den Klemmteilen 43, 44 eingeklemmt ist. Weiter ist in dem in Figur 3 hinteren Kulissenträger 41 ein Schlitz 42 für das Einschieben des Schweissmessers 4 zwischen die beiden Klemmteile 43, 44 vorhanden (in Figur 3 nicht sichtbar).

In der Figur 3 sind auch weitere Teile einer Heizung für das Schweissmesser 4 sichtbar, welche die bereits erwähnte Transformatoreinheit 90 umfasst. Um einen hohlzylindrischen Ferritkern dieser Einheit ist über dessen Umfang verteilt eine Primärwicklung durch mehrere Windungen Kupferdraht, beispielsweise 5-15 Windungen, gewickelt, wobei sich die einzelnen Windungen im Wesentlichen in Axialrichtung des Ferritkerns sowie durch diesen hindurch erstrecken. Die Sekundärwicklung wird demgegenüber nur durch einen einzelnen Leiter gebildet, der sich gerade durch den Ferritkern hindurch erstreckt und endseitig jeweils mit den unteren Abschnitten der Klemmarme 441 des Klemmteils 44 verschraubt ist (Schraube 901). Die Klemmarme 441 sind elektrisch leitend und bilden Teile des Sekundärkreises, der durch das ebenfalls elektrisch leitende Schweissmesser 4 geschlossen wird, sobald dieses zwischen dem ersten 43 und dem zweiten Klemmteil 44 eingeklemmt und mit den Klemmarmen 441 in Kontakt ist. Da das Schweissmesser 4 gegenüber dem die Sekundärwicklung bildenden geraden Leiter und den beiden Klemmarmen 441 einen wesentlich geringeren Querschnitt und einen höheren spezifischen Widerstand aufweist, ist sein elektrischer Widerstand vergleichsweise hoch, so dass in ihm der Grossteil der elektrischen Verlustleistung des Sekundärkreises anfällt. Zudem ergibt sich durch das Übersetzungsverhältnis der Transformatoreinheit 90 mit der lediglich einen Windung im Sekundärkreis ein hoher Sekundärstrom, so dass das Schweissmesser sehr effektiv und schnell innerhalb von 3 bis 4 Sekunden auf die gewünschte Temperatur von ca. 300 °C aufgeheizt wird. Ströme grösser als 100 A können hierbei erreicht werden.

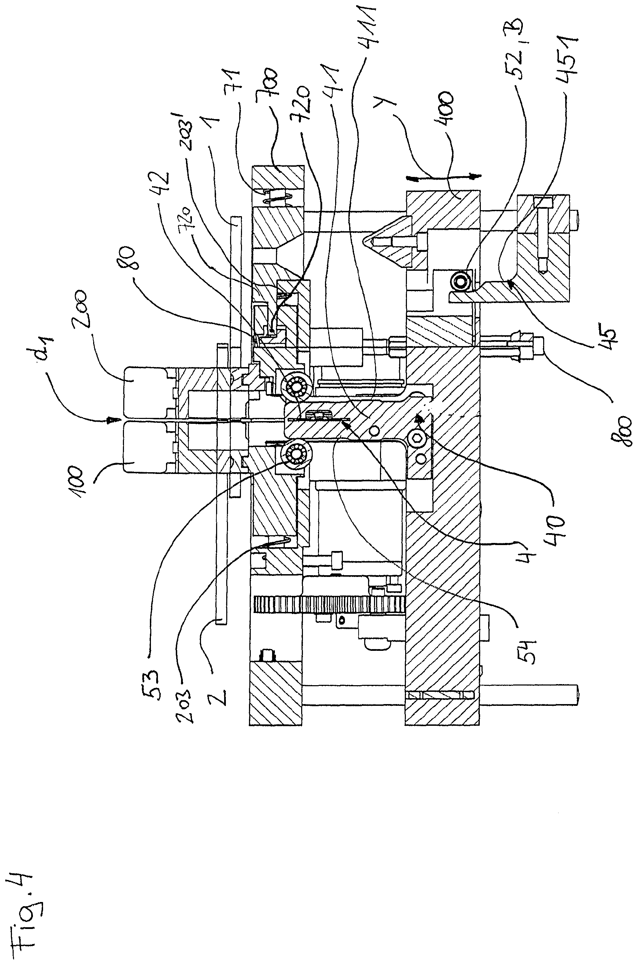

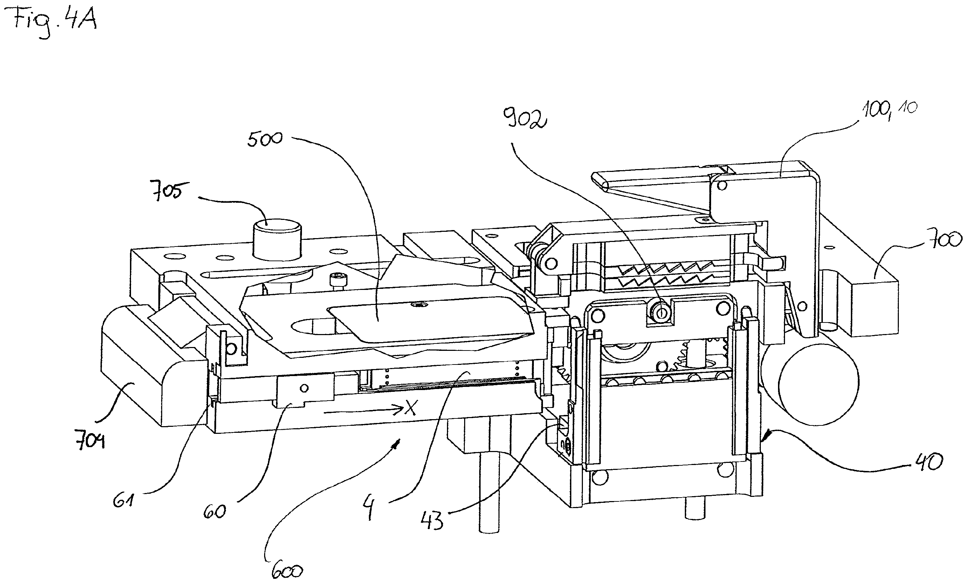

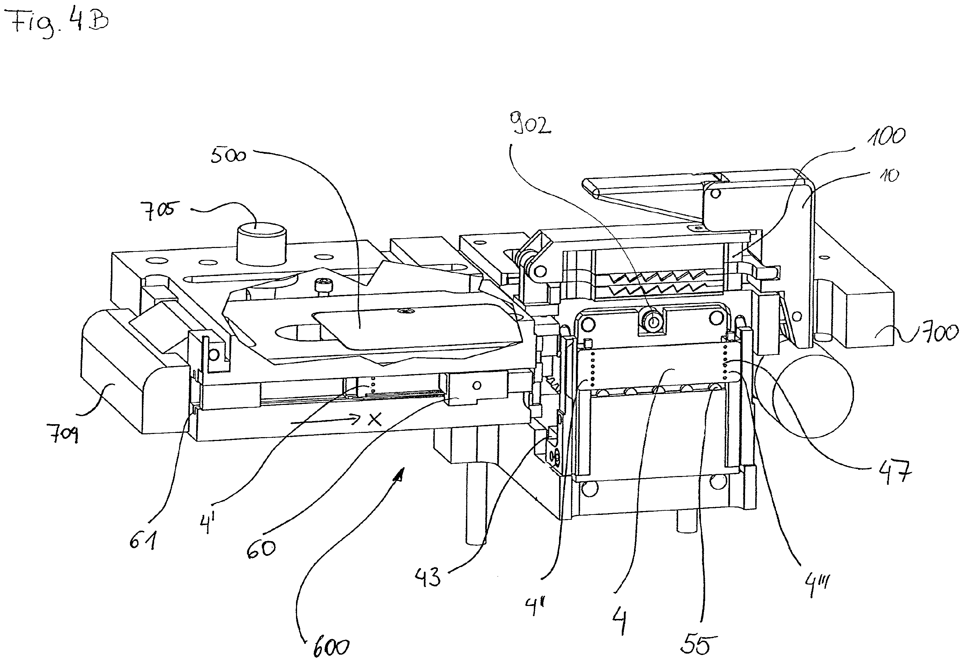

**Figur 4** zeigt eine Schnittdarstellung der erfindungsgemässen Vorrichtung, wobei sich die Plattform 400 und mit ihr der Schweissmesserhalter 40, die ja in vertikaler Richtung des Pfeils Y zum Tisch 700 bewegbar ist, in der sogenannten "Bladefeed Position" befindet. In dieser Position befindet sich der Schweissmesserhalter 40 auf einer Höhe, so dass das Schweissmesser 4 durch den Schlitz 42 zwischen die beiden Klemmteile 43, 44 eingeschoben werden kann. Dazu sind die beiden Klemmteile 43, 44 etwas voneinander beabstandet. Der Abstand der beiden Klemmteile 43, 44 voneinander wird entgegen der Wirkung der Federn 51 durch Abtasten einer zweiten Kulisse 451 an einem zweiten Kulissenträger 45 mit Hilfe eines Abtastrades 52 eingestellt. In der "Bladefeed Position" befindet sich das Abtastrad 52 an der zweiten Kulisse 451 in einer Aufnahmeposition (B), in der die beiden Klemmteile 43, 44 gerade den für das Einschieben des Schweissmessers 40 nötigen Abstand voneinander haben.

Die beiden Schlauchhalter 100, 200 sind im Tisch 700 jeweils verschiebbar montiert und somit nicht lediglich gegeneinander, sondern auch in gleicher Richtung miteinander. Durch zweite Federn sind sie gegeneinander vorgespannt. Die entsprechenden zweiten Federn sind in Figur 4 mit 203, 203' bezeichnet. An den Schlauchhaltern 100, 200 ist weiter jeweils ein Paar von Abtasträdern 53 gelagert. Die dadurch insgesamt vier Abtasträder 53 wirken mit den erwähnten vier ersten Kulissen 411 an den Kulissenträgern 41 zusammen, wenn diese, wie in der in Figur 4 dargestellten "Bladefeed Position", zwischen die Abtasträderpaare 53 eingreifen. Durch das Abtasten der vier ersten Kulissen 411 wird der horizontale Abstand d1 zwischen den beiden Schlauchhaltern 100, 200 eingestellt. In der in Figur 4 dargestellten "Bladefeed Position" befinden sich der erste und der zweite Schlauchhalter 100, 200 im Abstand d1 zu einander. Indem die vier ersten Kulissen 411 identisch und spiegelsymmetrisch zur Ebene des Schweissmessers 4 ausgebildet sind und die beiden Schlauchhalter 100, 200 in gleicher Richtung miteinander verschiebbar sind, werden die Schlauchhalter 100, 200 relativ zum Schweissmesserhalter 40 stets so ausgerichtet, dass das Schweissmesser sich genau in der Mitte zwischen den beiden Schlauchhaltern 100, 200 befindet. Allfällige Toleranzen zwischen der Plattform 400, die den Schweissmesserhalter 40 trägt, und dem Tisch 700, auf dem die Schlauchhalter 100, 200 montiert sind, werden dadurch ausgeglichen.

**Figur 4A** zeigt einen Teil der erfindungsgemässen Vorrichtung. Dargestellt wird einer der beiden Schlauchhalter 100 mit dem Spannhebel 10, angeordnet am Tisch 700. Die zweite Hälfte der Vorrichtung, umfassend den zweiten Schlauchhalter 200 und das zweite Klemmteil 44, ist in Figur 4A weggelassen. Teilweise sichtbar ist dadurch der Schweissmesserhalter 40 mit dem ersten Klemmteil 43. Der Schweissmesserhalter 40 befindet sich, wie in Fig. 4 beschrieben, in der "Bladefeed Position". Die Schläuche der ersten und zweiten Schlauchgruppe 1, 2 sollten in dieser Position an sich bereits in die Vorrichtung eingelegt sein, wobei sie in Figur 4 jedoch ebenfalls weggelassen sind. Die Schweissmesserzuführung 600 umfasst den Schieber 60, welcher mit dem Schlitten 601 entlang von Führungsschienen 61 bewegt und mit Hilfe eines weiteren Antriebs 602 angetrieben wird (vgl. Figur 1A). Der Schieber 60 wird zur Einführung des Schweissmessers 4 in den Schweissmesserhalter 40 in Richtung des Pfeils X bewegt und nimmt dabei das Schweissmesser 4 mit. Pro Schweissvorgang wird ein neues Schweissmesser eingeführt, sodass jedes Schweissmesser 4 nur einmal benutzt wird. Eine grössere Anzahl an Schweissmessern 4 ist in der Box 500 bevorratet. Die Box 500 ist derart ausgestaltet, dass pro Schweissvorgang jeweils ein Schweissmesser 4 in den Bereich der Führungsschienen 61 vorgeschoben wird, so dass es durch den Schieber 60 aufgegriffen und in den Schweissmesserhalter 40 verschoben werden kann. Ebenfalls in Fig. 4A ersichtlich ist ein Temperatursensor 902, mit welchem die Temperatur des Schweissmessers erfasst und kontrolliert wird.

**Figur 4B** zeigt die Teildarstellung aus Figur 4A, wobei das Schweissmesser 4 in den Schweissmesserhalter 40 eingeschoben ist. Ein weiteres Schweissmesser 4' ist für den nächsten Schweissvorgang bereits vorpositioniert. Im Schweissmesserhalter 40 wird das Schweissmesser 4 auf halbmondförmigen Schienen 55 geführt. Zum vollständigen Einführen des Schweissmessers 4 greift der Schieber 60 selbst ein Stück weit in den Schweissmesserhalter 40 ein. Bevor die weiteren Schritte mit vertikaler Verschiebung des Schweissmesserhalters 40 aus der "Bladefeed Position" ausgeführt werden können, muss der Schieber 60 daher unter Umkehr seiner Bewegungsrichtung zunächst aus dem Bereich des Schweissmesserhalters 40 wieder herausgefahren werden.. Figur 4B zeigt den Schieber 60 bereits schon ein Stück weit wieder zurückgefahren.

Figur 4B zeigt das Schweissmesser 4 in einer bevorzugten Ausführungsform. Entlang seiner beiden kürzeren Seiten des Rechtecks verlaufen Randbereiche 4" und 4"'. Im eingesetzten Zustand werden diese Randbereiche 4" und 4'" durch das erste und das zweite Klemmteil 43, 44 kontaktiert (nicht sichtbar). Für eine gute elektrische Kontaktierung weist das Klemmteil 44 an der Innenseite der aufragenden Abschnitte der Klemmarme 441 hierzu vorspringende Kontaktpunkte, vorzugsweise in Form federnder Druckstücke, auf. In der Nähe der Randbereiche weist das Schweissmesser 4 Ausnehmungen 48 auf, welche als Bohrungen entlang der beiden kurzen Rechteckseiten ausgestaltet sind. Entlang seiner unteren Rechteckseite liegt das Schweissmesser 4 punktuell auf den halbmondförmigen Schienen 55 auf.

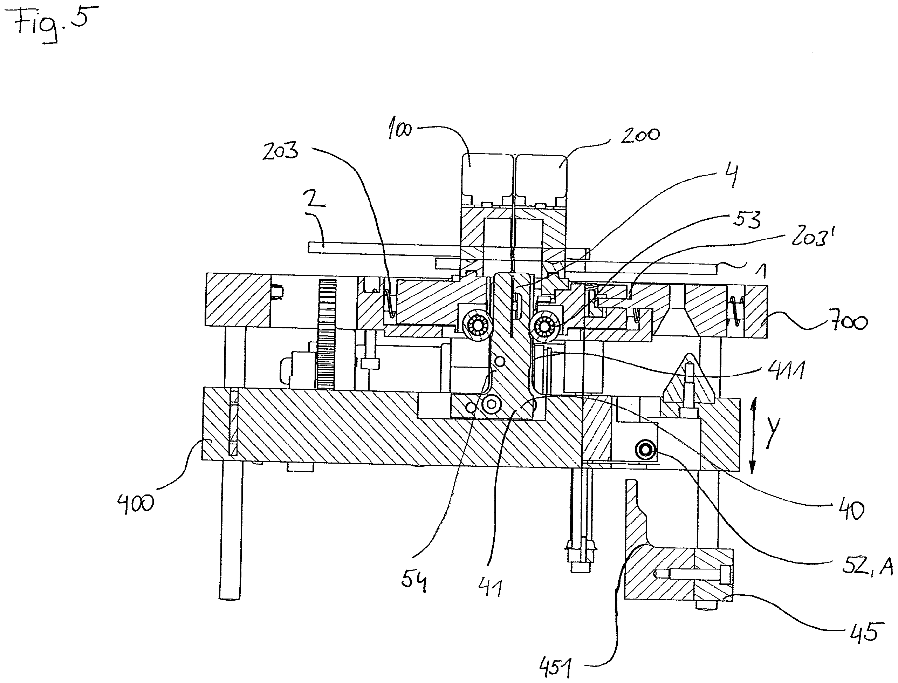

**Figur 5** zeigt eine Schnittdarstellung der erfindungsgemässen Vorrichtung, wobei sich die Plattform 400, mit Hilfe derer der Schweissmesserhalter 40 in vertikaler Richtung des Pfeils Y bewegt wird, in der sogenannten "Pre-cut Position" befindet. Gegenüber und ausgehend von der "Bladefeed Position" gemäss Figur 4 ist die Plattform 400 ein Stück weit nach oben verschoben. In dieser Position befindet sich nun das Abtastrad 52 oberhalb des zweiten Kulissenträgers 45 ausser Eingriff mit der zweiten Kulisse 451 in einer sogenannten Kontaktierungsposition (A). In dieser Position werden die beiden Klemmteile 43, 44 nicht länger durch die zweite Kulisse 451 auf Abstand voneinander gehalten, sondern können nunmehr das Schweissmesser 4 zwischen sich einklemmen, wobei es gleichzeitig elektrisch kontaktiert wird. In dieser Position erfolgt dann auch die elektrische Beheizung des Schweissmessers 4.

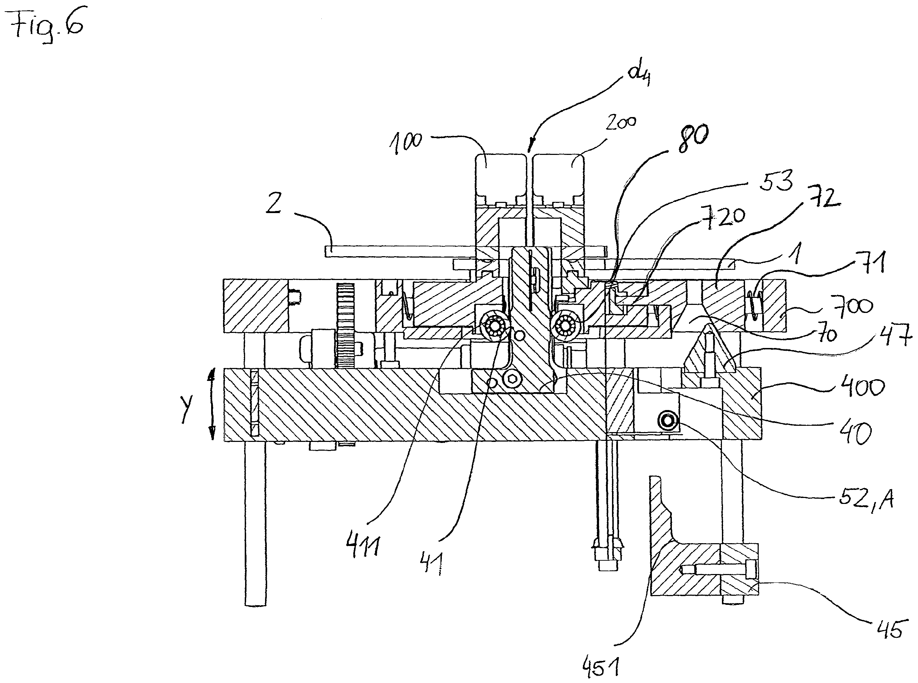

**Figur 6** zeigt eine Schnittdarstellung der erfindungsgemässen Vorrichtung, wobei sich die Plattform 400, mit Hilfe derer der Schweissmesserhalter 40 in vertikaler Richtung des Pfeils Y bewegt wird, in der sogenannten "Stretch Position" befindet. Verglichen mit Figur 4 und Figur 5 wurde die Plattform 400 weiter in Richtung des Tisches 700 bewegt und die Abtasträder 53 befinden sich nunmehr an den ersten Kulissen 411 in einer Position, in der der Abstand der beiden Schlauchhalter 100, 200 den grössten Wert d4 annimmt. Die Schläuche der ersten und der zweiten Schlauchgruppe 1, 2 werden dadurch gedehnt. Die ersten Kulissen 411 angeordnet an den Kulissenträgern 41 sind so ausgebildet, dass dieser Abstand d4 bei der folgenden Weiterbewegung der Plattform 400 in

Richtung des Tisches 700, während der das Schneidmesser 4 die Schläuche durchtrennt, erhalten bleibt. Die Schläuche werden dadurch im gedehnten Zustand durchtrennt.

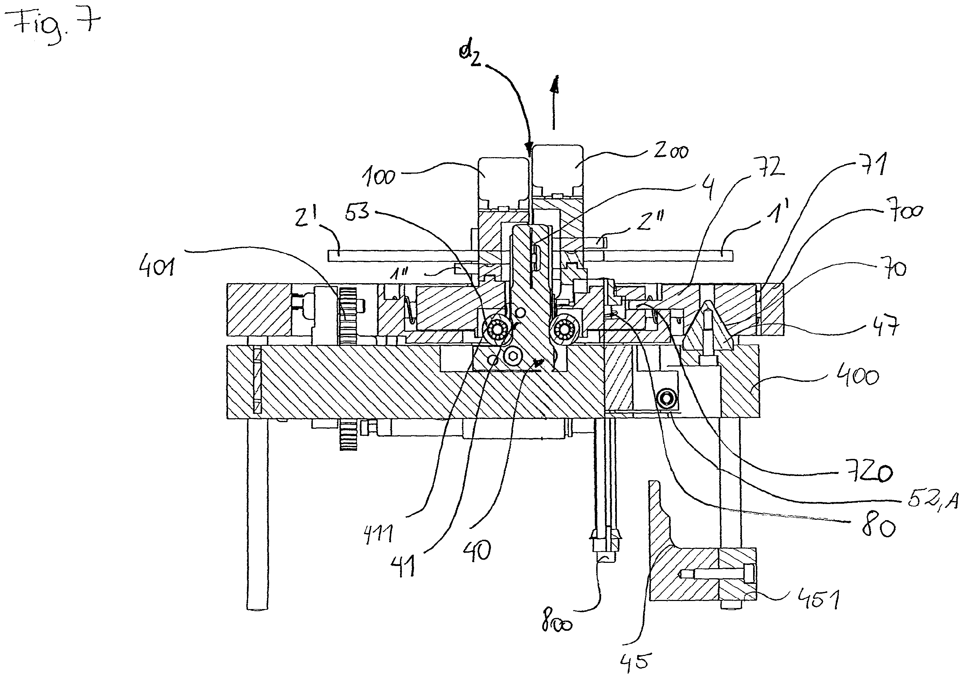

**Figur 7** zeigt eine Schnittdarstellung der erfindungsgemässen Vorrichtung, wobei sich die Plattform 400 in der sogenannten "Cut Position" befindet, in welcher die Schläuche der ersten und der zweiten Schlauchgruppe 1, 2 durchtrennt sind.

Unmittelbar nach dem Durchtrennen der Schläuche müssen die Schnitt-Enden 1' der Schläuche der ersten Schlauchgruppe auf die Schnitt-Enden 2' der Schläuche der zweiten Schlauchgruppe in der Höhe ausgerichtet werden. Dazu ist der zweite Schlauchhalter 200 vertikal verschiebbar und mittels einer dritten Feder 80 nach oben vorgespannt. Diese Bewegung des zweiten Schlauchhalters 200 ist in den soweit beschriebenen Positionen jedoch blockiert durch den Eingriff eines Anschlagsfläche 720 an einem Riegel 72, der im Tisch 700 horizontal verschiebbar und von einer vierten Feder 71 gegen den zweiten Schlauchhalter vorgespannt ist. Die Entriegelung des zweiten Schlauchhalters 200 durch Zurückziehen der Anschlagsfläche 720 erfolgt durch Eingriff eines an der Plattform 400 angebrachten Kegels 47 in einen exzentrisch zu diesem angeordneten Hohlkegel 70 auf der Unterseite des Riegels 72. Die vierte Feder 71 wird dabei zusammengedrückt. Wie dies in Figur 6 bereits erkennbar ist, beginnt sich in der "Stretch Position" der Kegel 47 bereits in den Hohlkegel 70 einzuschieben. Der Kegel 47 und der Hohlkegel 70 sind so aufeinander abgestimmt, dass die Verriegelung des zweiten Schlauchhalters 200 bei der vertikalen Bewegung der Plattform 400 gelöst wird, sobald die Schläuche beider Schlauchgruppen 1, 2 vollständig durchtrennt sind. Nach dem Lösen der Verriegelung drückt die dritte Feder 80 den zweiten Schlauchhalter 200 nach oben.

Wie in Figur 7 dargestellt, greift in der "Cut-Position" der Kegel 47 vollständig in den Hohlkegel 70 ein und hat die Verriegelung des zweiten Schlauchhalters 200 gelöst, so dass dieser durch die dritte Feder 80 nach oben verschoben werden konnte. Dadurch sind die Schnitt-Enden 1' der Schläuche der ersten Schlauchgruppe auf die Schnitt-Enden 2' der Schläuche der zweiten Schlauchgruppe in der Höhe ausgerichtet. Das Schweissmesser 4 befindet sich noch zwischen den Schläuchen.

In der "Cut Position" hat die Plattform 400 ihre höchste Position erreicht. Das Abtastrad 52 der zweiten Kulisse 451 befindet sich weiterhin oberhalb von dieser in der Kontaktierungsposition (A), so dass das Schweissmesser 4 zwischen den Klemmteilen 43, 44 eingeklemmt ist und beheizt werden kann und auch wird. Über die Abtasträder 53 wird an den ersten Kulissen 411 der Abstand der Schlauchhalter 100, 200 zu einander auf den Abstand d2 eingestellt, der so bemessen ist, dass die Schnitt-Enden 1' und 2' sowie die Rest-Enden 1", 2" an das heisse Schweissmesser 4 gedrückt und dadurch aufgeschmolzen werden.

Danach wird das Schweissmesser 4 aus dem Bereich der geschnittenen Schläuche herausgefahren. Dies erfolgt, indem die Plattform 400 und mit ihr der Schweissmesserhalter 40 nunmehr nach unten bewegt wird. Die Umkehr ihrer Bewegungsrichtung erfolgt ohne Umkehr der Drehrichtung des Motors 404 des Antriebs 401 automatisch durch das Pleuel 405. Dabei passieren die Abtasträder 53 an den ersten Kulissen 411 zunächst wieder den Bereich, den sie während der "Stretch Position" bereits überfahren haben, während dessen der Abstand der beiden Schlauchhalter 100, 200 den grösseren Wert d4 aufweist. Die Schläuche mit ihren aufgeschmolzenen Enden werden dadurch vom Schweissmesser 4 etwas wegbewegt, was dessen Herausfahren erleichtert. Ferner wird kein Schmelzmaterial, welches sich an den aufgeschmolzenen Enden befindet durch das Herausfahren abtransportiert und steht für das anschliessende Verschweissen zur Verfügung.

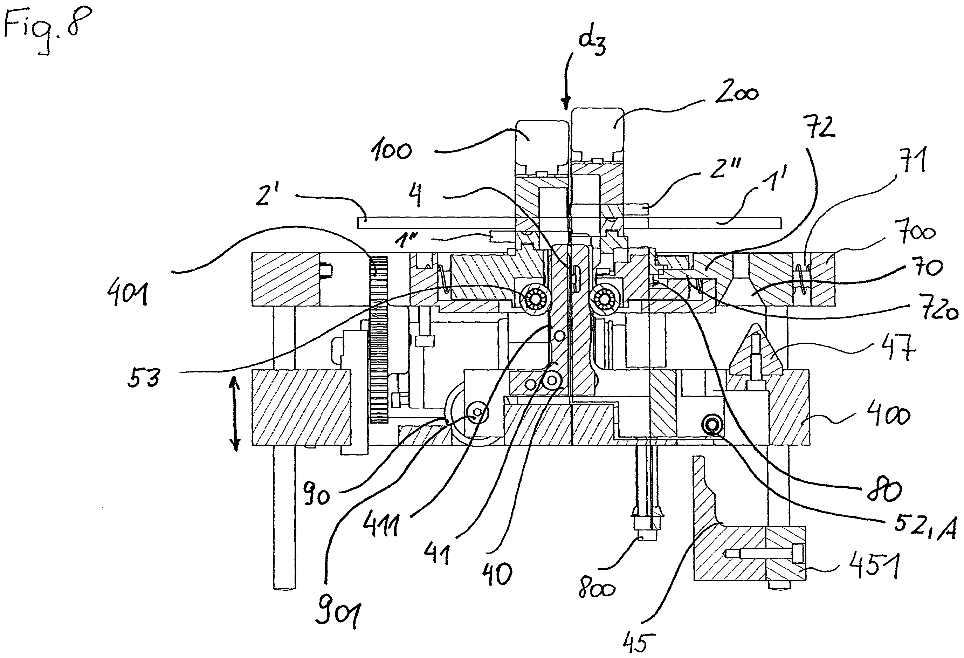

Bei der Weiterbewegung der Plattform 400 nach unten kommen die Abtasträder 53 an den ersten Kulissen 411 wieder in den Bereich, den sie während der "Precut Position" überfahren haben und während dem der Abstand der beiden Schlauchhalter 100, 200 den kleineren Wert d3 aufweist. Hierdurch kommt je ein Schnitt-Ende 1' der ersten Schlauchgruppe mit je einem Schnitt-Ende 2' der zweiten Schlauchgruppe in Berührung und wird zu durchgängigen Schläuchen verschweisst. Diese sogenannte "Press-Position" zeigt **Figur 8****.** In der "Press-Position von Fig. 8 bleibt die Plattform 400 stehen, so dass die gefügten Schläuche sowie die Rest-Enden 1", 2", nach einer Abkühlung, aus der Vorrichtung entnommen werden können.

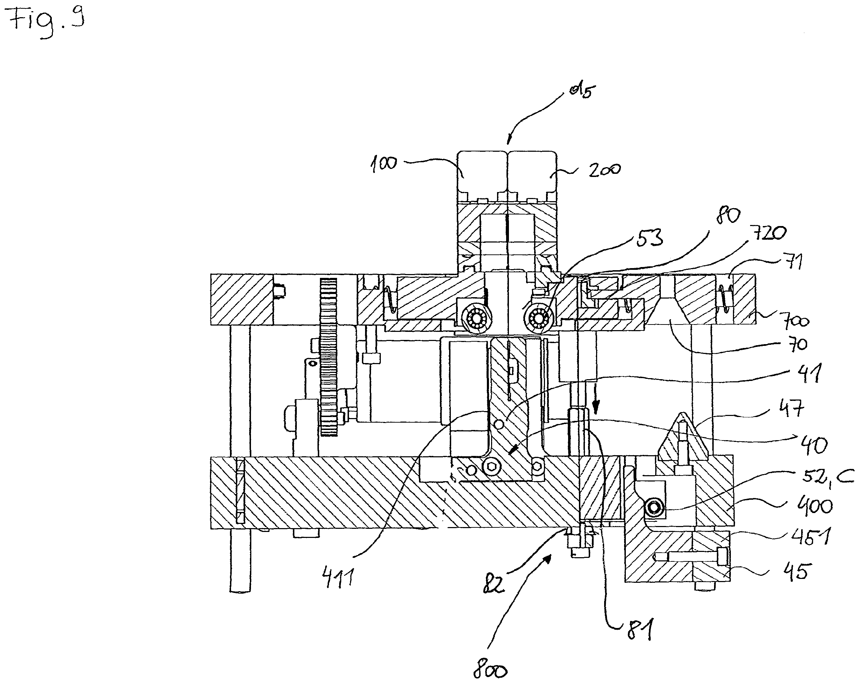

**Figur 9** zeigt eine Schnittdarstellung der erfindungsgemässen Vorrichtung, mit der Plattform 400 und dem Schweissmesserhalter 40 in der "Eject Position". In der "Eject Position" befindet sich die Plattform 400 in ihrer tiefsten Position. Die verschweissten Schläuche und die Rest-Enden wurden bereits aus den Schlauchhaltern 100, 200 entnommen. Wie bereits zu Figur 4 erwähnt, wird der Abstand der beiden Klemmteile 43, 44 zu einander durch Abtasten der zweiten Kulisse 451 mit Hilfe eines Abtastrades 52 eingestellt. Ist der Schweissmesserhalter 40 in der "Eject Position", so befindet sich das Abtastrad 52 der zweiten Kulisse 451 in einer Freigabeposition (C). Das erste Klemmteil 43 wurde dabei entgegen der Wirkung der ersten Federn 51 (in Figur 9 nicht sichtbar) relativ zum zweiten Klemmteil 44 so bewegt, sodass die Spaltbreite zwischen den beiden Klemmteilen 43, 44 auf maximale Distanz geöffnet ist. Das Schweissmesser 4 ist dann frei und kann aus dem Schweissmesserhalter 40 in eine Entsorgungsbox (in Figur 9 nicht dargestellt) nach unten herausfallen.

Beim Übergang von der "Press Position" gemäss Fig. 8 in die "Eject Position" gemäss Fig. 9 wird der zweite Schlauchhalter 200 aus seiner nach oben verschobenen Stellung in seine Ausgangsposition auf gleicher Höhe mit dem ersten Schlauchhalter 100 zurückbewegt. Gleichzeitig wird die dritte Feder 80 gespannt. Dazu dient ein Rückholmechanismus 800 mit einer mit dem zweiten Schlauchhalter 200 gekoppelten Zugstange 81. Beim nach unten Fahren schlägt die Plattform 400 an einem Anschlag 82 der Zugstange 81 an und zieht diese mit nach unten. Auch der Kegel 47 kommt dabei ausser Eingriff mit dem Hohlkegel 70, so dass die Anschlagsfläche 720 wie zuvor in den zweiten Schlauchhalter 200 einrasten. Der zweite Schlauchhalter befindet sich in seiner Ausgangsposition auf gleicher Höhe mit dem ersten Schlauchhalter 100.

Beim nach unten Fahren in die "Eject Position" kommen schliesslich die Abtasträder 53 ebenfalls ausser Eingriff Kontakt mit den ersten Kulissen 411 an den Kulissenträgern 41. Der Abstand zwischen den beiden Schlauchhaltern 100, 200 verringert sich dabei soweit, dass die Schlauchhalter 100, 200 einander berühren.

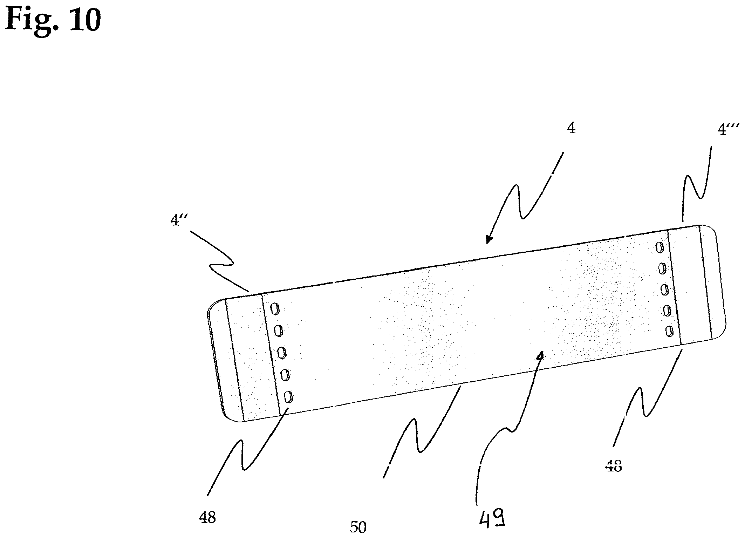

**Figur 10** zeigt die bevorzugte Ausführungsform des als solches nicht beanspruchten Schweissmessers 4 in einer rechteckigen Form mit Ausnehmungen 48, angeordnet jeweils in einer Reihe entlang der Randbereiche 4", 4'" der beiden kürzeren Rechtecksseiten. Das Schweissmesser 4 ist vorzugsweise durch hälftige Längsfaltung eines Blättchens von doppelter Breite hergestellt. Als Klinge 50, welche die Schläuche schneidet, dient die Biegekante entlang von welcher die beiden Hälften des Blättchens zusammenhängen. Alternativ zur Faltung wird das Schweissmesser 4 durch Stanzen und dessen Klinge 50 durch Prägung hergestellt. Durch diese Gestaltung wird in rationeller Weise eine nicht zu scharfe, sondern abgerundete, vor allem jedoch gleichmässige und zu beiden Seiten hin symmetrische Schneidkante geschaffen. Letzteres ist wichtig, damit das Schneidmesser beim Durchtrennen der Schläuche nicht zu einer Seite hin abgelenkt wird, wie dies beispielsweise der Fall bei einer unsymmetrisch geschliffenen Kante wäre.

Die Klinge 50, welche die Schläuche schneidet, verläuft entlang einer der beiden längeren Rechtecksseiten.

Das Schweissmesser 4 weist beispielsweise eine Länge zwischen 60-80 mm, insbesondere von 70 mm, eine Breite zwischen 15-20 mm, insbesondere von 17 mm und eine Stärke zwischen 0.2-0.5 mm, insbesondere von 0.3 mm auf. Die Grösse der Schmelzfläche 49 hängt in erster Linie vom Durchmesser der Schläuche und von der Anzahl der zu verschweissenden Schläuche ab.

Beim verwendeten Material für das Schweissmesser 4 handelt es sich beispielsweise um Chrom/Nickelstahl.

Für ein qualitativ hochwertiges Schweissergebnis relevant ist, die horizontale sowie die vertikale Temperaturverteilung über die Fläche des Schweissmessers. Es wurde festgestellt, dass die Anordnung der Ausnehmungen entlang der Randbereiche 4", 4'" zu einer homogeneren vertikalen Wärmeverteilung führt. Die gewünschte Temperatur des Schweissmessers beträgt ca. 300 °C.

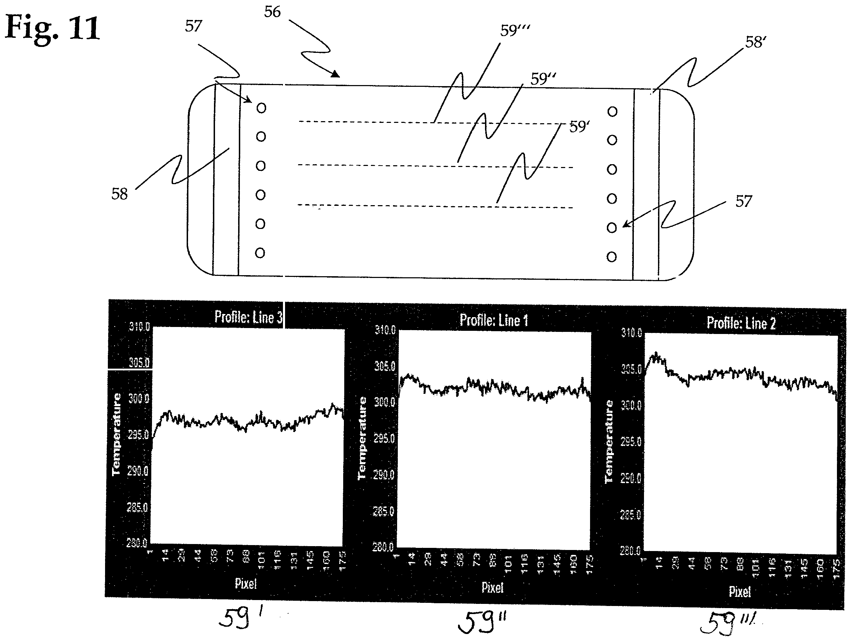

**Figur 11** zeigt eine weitere Ausführungsform eines als solches nicht beanspruchten Schweissmessers 56. Hier sind pro Randzone 58, 58' beispielsweise je sechs, vorzugsweise kreisrunde Ausnehmungen 57 angeordnet. Die Anzahl sowie die geometrische Form dieser Ausnehmung ist nicht auf die dargestellte Anzahl und eine kreisrunde Form beschränkt. Das Verhältnis zwischen Stegbreite zu Schlitzbreite bzw. Durchmesser beträgt in der gezeigten Ausführungsform 60 %. Die darunterliegende Darstellung zeigt das Messprotokoll mit der horizontalen Temperaturverteilung entlang von drei strichlierten Linien 59', 59", 59"'. Abgelesen aus den Messprotokollen kann ebenfalls die vertikale Temperaturverteilung werden. Die Linien 59', 59", 59'" wurden in einer Höhe gewählt, in welcher in der erfindungsgemässen Vorrichtung später die Schläuche durchlaufen werden.

Dem Protokoll liegen Aufnahmen mit einer Wärmebildkamera zugrunde. In diesem Protokoll wird die horizontale Temperaturverteilung entlang der Linien 59', 59", 59'" gezeigt. Das Profil "Linie 3" entspricht der Messung entlang der Linie 59'. Das Profil "Linie 1", jenem entlang der Linie 59" und das Profil "Linie 2" jenem entlang der Linie 59"'. Die auf der y-Achse angegebenen Werte der Pixel entsprechen horizontalen Längeneinheiten entlang der Linien 59', 59", 59"'.

Das als solches nicht beanspruchte Schweissmesser 56 wurde für die Messung der Temperaturverteilung in den Schweissmesserhalter 40 umfassend das erste und das zweite Klemmteil 43,44 eingespannt und für die Messung geschwärzt. Das Schweissmesser schliesst den Sekundärstromkreis der Transformatoreinheit und wird bedingt durch seinen hohen Widerstandswert auf eine Solltemperatur von ca. 300 °C erhitzt. Es konnte festgestellt werden, dass die optimale Temperaturverteilung (300 °C +/- 5 °C) innert 3-4 Sekunden erreicht war. Bei den Versuchen konnte des weiteren herausgefunden werden, dass die Kontaktierung des Schweissmessers durch die beiden Klemmteile einen Einfluss auf die vertikale Temperaturverteilung hat. Eine symmetrische Verteilung der Klemmkraft entlang der beiden Randzonen hat sich als relevant für die vertikale Temperaturverteilung erwiesen. Eine Möglichkeit hierfür sind vorzugsweise federnde Druckstücke angeordnet auf mindestens einem der beiden Klemmteile. Mit Hilfe dieser Druckstücke wird eine symmetrische Kontaktierung erzielt, indem die Druckkraft aller Druckstücke in etwa gleich eingestellt wird.

### BEZEICHNUNGSLISTE

- 1: Schläuche der ersten Schlauchgruppe
- 1': Schnitt-Enden der Schläuche der ersten Schlauchgruppe
- 1": Rest-Enden der Schläuche der ersten Schlauchgruppe
- 2: Schläuche der zweiten Schlauchgruppe
- 2': Schnitt-Enden der Schläuche der zweiten Schlauchgruppe
- 2": Rest-Enden der Schläuche der zweiten Schlauchgruppe
- 100: Erster Schlauchhalter
- 101: erste Schlauchklemme des ersten Schlauchhalters
- 102: zweite Schlauchklemme des ersten Schlauchhalters
- 51: erste Feder
- 11: Durchgangsöffnungen durch erste Schlauchklemme zweiter Schlauchhalter
- 12: Durchgangsöffnungen durch zweite Schlauchklemme erster Schlauchhalter
- 200: zweiter Schlauchhalter
- 201: erste Schlauchklemme des zweiten Schlauchhalters
- 202: zweite Schlauchklemme des zweiten Schlauchhalters
- 203, 203': zweite Federn
- 21: Durchgangsöffnungen durch erste Schlauchklemme zweiten Schlauchhalters
- 22: Durchgangsöffnungen durch zweite Schlauchklemme zweiten Schlauchhalters
- 31: erste Quetschzone
- 32: zweite Quetschzone
- 4: Schweissmesser
- 4': weiteres Schweissmesser
- 4", 4"': Randbereiche
- 40: Schweissmesserhalterung
- 41: ein Paar von Kulissenträgern
- 42: Schlitz
- 43: erstes Klemmteil
- 44: zweites Klemmteil
- 45: zweiter Kulissenträger
- 47: Kegel
- 48: Ausnehmungen
- 49: Schmelzfläche
- 50: Klinge
- 51: erste Federn
- 52: Abtastrad der zweiten Kulisse
- 53: Abtastradpaar der ersten Kulisse
- 55: halbmondförmige Schienen
- 56: Schweissmesser
- 57: kreisrunde Ausnehmungen
- 58, 58': Randzone
- 59', 59", 59'": drei strichlierten Linien
- 400: Plattform zur Positionierung der Schweissmesserhalterung
- 401: Antrieb für die Plattform
- 402, 403, 407: Zahnräder
- 404: Motor
- 405: Pleuel
- 406: stangenförmige Führung
- 411: vier erste Kulissen
- 431, 441: abgewinkelte Klemmarme
- 451: zweite Kulisse
- 500: Box mit Schweissmessern
- 520: stangenförmige Führung
- 600: Schweissmesserzuführung
- 60: Schieber
- 61: Führungsschienen
- 601: Schlitten
- 602: Antrieb für Schweissmesserzuführung
- 603: Zahnriemen
- 700: Tisch
- 70: Hohlkegel
- 71: vierte Federn
- 72: Riegel
- 720: Anschlagsfläche
- 701: vier Beine
- 702: Ausschnitt
- 704: Schublade
- 705: Startknopf
- 10,20: Spannhebel
- 800: Rückholmechanismus
- 80: dritte Feder
- 81: Zugstange
- 82: Anschlag
- 90: Transformatoreinheit
- 901: Schraube
- 902: Temperatursensor

Kontaktierungsposition (A)
Aufnahmeposition (B)
Freigabeposition (C)

## Patentansprüche

1. Vorrichtung zum Verschweissen von thermoplastischen Schläuchen einer ersten und zweiten Schlauchgruppe (1, 2), umfassend zwei Schlauchhalter (100, 200) mit je einer ersten (101, 201) und einer zweiten Schlauchklemme (102, 202) sowie ein Schweissmesser (4),
wobei die Schläuche der ersten Schlauchgruppe (1) gerade durchlaufend zwischen den beiden Schlauchhaltern (100, 200) in Durchgangsöffnungen (11, 21) der beiden ersten Schlauchklemmen (101, 201) eingelegt und darin gequetscht werden können,
wobei die Schläuche der zweiten Schlauchgruppe (2) gerade durchlaufend zwischen den beiden Schlauchhaltern (100, 200) in Durchgangsöffnungen (12, 22) der beiden zweiten Schlauchklemmen (102, 202) eingelegt und darin gequetscht werden können,
wobei das Schweissmesser (4) zwischen dem ersten und dem zweiten Schlauchhalter (100, 200) bewegt werden kann und dazu eingerichtet ist, dabei die gequetschten Schläuche der ersten und der zweiten Schlauchgruppe (1, 2) in einer Bewegung zu durchtrennen um auf diese Weise Schnitt-Enden (1', 2') und Rest-Enden (1", 2") zu erzeugen,
wobei einer der beiden Schlauchhalter (100, 200) relativ zum anderen Schlauchhalter (100, 200) verschoben werden kann, um dadurch die Schnitt-Enden (1', 2') der Schläuche aufeinander auszurichten, und
wobei die beiden Schlauchhalter (100, 200) in einer horizontalen Richtung zu einander oder weg von einander verschoben werden können, um dadurch je ein Schnitt-Ende (1') eines Schlauchs der ersten Schlauchgruppe (1) mit je einem Schnitt-Ende (2') eines Schlauchs der zweiten Schlauchgruppe (2) zu je einem durchgängigen Schlauch gleichzeitig zu verschweissen, **dadurch gekennzeichnet, dass** die Durchgangsöffnungen (11, 21) der beiden ersten Schlauchklemmen (101, 201) auf einer ersten Ebene und die Durchgangsöffnungen (12, 22) der beiden zweiten Schlauchklemmen (102, 202) auf einer zweiten Ebene angeordnet sind, wobei die erste Ebene parallel und beabstandet zur zweiten Ebene angeordnet ist.

2. Vorrichtung nach Anspruch 1, **dadurch gekennzeichnet, dass** die beiden ersten Schlauchklemmen (101, 201) und die beiden zweiten Schlauchklemmen (102, 202) Quetschzonen (31, 32) aufweisen, wobei diese Quetschzonen (31, 32) entlang der Durchgangsöffnungen (11, 21, 12, 22) verlaufen und dazu eingerichtet sind, die eingelegten Schläuche in einem schrägen Winkel zur ersten bzw. zur zweiten Ebene zu quetschen.

3. Vorrichtung nach einem der vorangegangenen Ansprüche, **dadurch gekennzeichnet, dass** das Schweissmesser (4) in einem Schweissmesserhalter (40) einklemmbar ist und eine Antriebseinheit (401) eingerichtet ist, den Schweissmesserhalter (40) zum Durchtrennen der Schläuche in einer vertikalen Richtung zu bewegen und dabei einer der beiden Schlauchhalter (100, 200) relativ zum anderen Schlauchhalter (100, 200) zum Ausrichten der Schnitt-Enden (1', 2') der Schläuche vertikal verschiebbar ist.

4. Vorrichtung nach Anspruch 3, **dadurch gekennzeichnet, dass** durch die Bewegung des Schweissmesserhalters (40) in vertikale Richtung ferner die beiden Schlauchhalter (100, 200) in der horizontalen Richtung, auf einander zu oder voneinander weg oder beide Schlauchhalter (100, 200) gleichzeitig in eine Richtung bewegbar sind, und vorzugsweise wobei die Vorrichtung eine in der vertikalen Richtung bewegbare Plattform (400) aufweist und der Schweissmesserhalter (40) auf dieser Plattform (400) angeordnet ist.

5. Vorrichtung nach einem der Ansprüche 3 bis 4, **dadurch gekennzeichnet, dass** der Schweissmesserhalter (40) zwei sich gegenüberliegende Klemmteile (43, 44) aufweist, zwischen welche das Schweissmesser (4) einklemmbar ist, vorzugsweise eines der beiden Klemmteile feststehend und das andere relativ beweglich zum feststehenden Klemmteil angeordnet ist.

6. Vorrichtung nach Anspruch 5, **dadurch gekennzeichnet, dass** an mindestens einem der beiden Klemmteile (43, 44) ein Paar von ersten Kulissenträgern (41) vorgesehen ist und das Paar von Kulissenträgern (41), vorzugsweise insgesamt vier erste Kulissen (411) aufweist, wobei diese Kulissen (411) untereinander identisch und spiegelsymmetrisch zu einer Ebene des Schweissmessers (4) angeordnet sind, wenn das Schweissmesser (4) zwischen die beiden Klemmteile eingeklemmt ist.

7. Vorrichtung nach Anspruch 6, **dadurch gekennzeichnet, dass** Abtasträderpaare (53) vorgesehen sind, bei der Bewegung des Schweissmesserhalters (40) in vertikaler Richtung die Kulissen (411) abzutasten und dabei die beiden Schlauchhalter (100, 200) in der horizontalen Richtung zu einander, oder weg von einander, oder beide Schlauchhalter (100, 200) gleichzeitig in eine Richtung bewegbar sind.

8. Vorrichtung nach einem der Ansprüche 5 bis 7, **dadurch gekennzeichnet, dass** ein zweiter Kulissenträger (45) und ein weiteres Abtastrad (52) vorgesehen sind, wobei das weitere Abtastrad (52) dazu eingerichtet ist, bei der Bewegung des Schweissmesserhalters (40) in vertikaler Richtung den zweiten Kulissenträger (45) abzutasten um dabei das eine Klemmteil (43) relativ zum zweiten feststehenden Klemmteil (44) zu bewegen und auf diese Weise der Abstand zwischen den beiden Klemmteilen (43, 44) variierbar ist.

9. Vorrichtung nach Anspruch 8, **dadurch gekennzeichnet, dass** das Abtasten des zweiten Kulissenträgers (45) durch das weitere Abtastrad (52) erfolgt, wobei drei unterschiedliche Positionen ermöglicht werden, nämlich eine erste Kontaktierungsposition (A) in welcher der Abstand zwischen den beiden Klemmteilen so gewählt ist, dass das Schweissmesser (4) eingeklemmt und gleichzeitig elektrisch kontaktiert ist, eine zweite Position (C) bei der der Abstand zwischen den beiden Klemmteilen (43, 44) so gewählt, dass das Schweissmesser ausgeworfen werden kann und eine Aufnahmeposition (B) bei der der Abstand zwischen den beiden Klemmteilen (43, 44) so gewählt ist, dass das Schweissmesser zwischen die beiden Klemmteile (43, 44) eingeschoben werden kann.

10. Vorrichtung nach Anspruch 9, **dadurch gekennzeichnet, dass** an mindestens einem der beiden Klemmteile (43, 44) Druckstücke angeordnet sind, welche das Schweissmesser (4) in der Kontaktierungsposition (A) kontaktieren, wobei diese Druckstücke an dem mindestens einen Klemmteil (43, 44) federnd gelagert sind.

11. Vorrichtung nach einem der Ansprüche 5 bis 10, **dadurch gekennzeichnet, dass** die Vorrichtung ferner eine Transformatoreinheit (90) mit einer Primärwicklung und einer Sekundärwicklung aufweist, wobei die Sekundärwicklung durch einen einzigen Leiter gebildet wird und dieser Leiter mit einem der beiden Klemmteile (43, 44), vorzugsweise mit dem feststehenden Klemmteil (44) verbunden ist, wobei das Schweissmesser (4) in der Kontaktierungsposition (A) den sekundären Stromkreis schliesst und auf diese Weise auf die gewünschte Temperatur aufgeheizt wird.

12. Vorrichtung nach einem der vorangegangenen Ansprüche, **dadurch gekennzeichnet, dass** die erste und die zweite Schlauchgruppe (1, 2) je die gleiche Anzahl an einzelnen Schläuchen, vorzugsweise mehr als zwei, insbesondere sechs Schläuche, pro Schlauchgruppe (1, 2) aufweisen.

13. Verfahren zum Verschweissen einer Vielzahl von Schläuchen einer ersten und einer zweiten Schlauchgruppe (1, 2) mit einer Vorrichtung nach einem der vorangegangenen Ansprüche 1 bis 12, wobei in einem Schritt die Schläuche der ersten und der zweiten Schlauchgruppe parallel und übereinander in sich gegenüberliegende erste (100) und zweite Schlauchhalter (200) gerade durchlaufend eingelegt und gequetscht werden und ein beheizbares Schweissmesser (4) in einen Schweissmesserhalter (4) eingelegt und auf eine gewünschte Temperatur erhitzt wird, das beheizte Schweissmesser (4) zwischen dem ersten und dem zweiten Schlauchhalter (100, 200) bewegt wird und dabei die gequetschten Schläuche gleichzeitig in Schnitt-Enden (1', 2') und Rest-Enden (1", 2") durchtrennt werden, die Schnitt-Enden (1', 2') der Schläuche durch das beheizte Schweissmesser (4) aufgeschmolzen werden,
die Schnitt-Enden (1') der Schläuche der ersten Schlauchgruppe (1) auf die Schnitt-Enden (2') der Schläuche der zweiten Schlauchgruppe (2) ausgerichtet werden, wobei einer der beiden Schlauchhalter (100, 200) relativ zum anderen Schlauchhalter (100, 200) verschoben wird, sodass die Schnitt-Enden (1') der Schläuche der ersten Schlauchgruppe (1) symmetrisch gegenüberliegend zu den Schnitt-Enden (2') der zweiten Schlauchgruppe (2) angeordnet sind,
die beiden Schlauchhalter (100, 200) aufeinander zu verschoben werden um dadurch je ein Schnitt-Ende (1') der Schläuche der ersten Schlauchgruppe (1) mit je einem Schnitt-Ende (2') der Schläuche der zweiten Schlauchgruppe (2) zu je einem durchgängigen Schlauch gleichzeitig verschweisst werden,
das Schweissmesser (4) aus dem Schweissmesserhalter (40) ausgeworfen wird.

14. Verfahren nach Anspruch 13, **dadurch gekennzeichnet, dass** beim Einlegen des Schweissmessers (4) der horizontale Abstand zwischen dem ersten und dem zweiten Schlauchhalter (100, 200) d1 ist, i beim Durchtrennen der gequetschten Schläuche der Abstand auf d4 vergrösserst wird, beim Aufschmelzen der Schnitt-Enden der Abstand d2 beträgt, wobei gilt d1 <d2<d4, beim Verschieben einer der beiden Schlauchhalter (100, 200) der Abstand wieder auf d4 vergrössert wird, beim Verschweissen der Abstand d3 ist, wobei gilt d3<d4 und beim Auswerfen des Schweissmessers der Schnitt-Enden (1', 2') der Abstand zwischen den beiden Schlauchhaltern (100, 200) d5 beträgt, wobei d5 den kleinsten aller fünf Abstände (d1, d2, d3, d4) darstellt.

## Claims

1. Device for sealing thermoplastic tubes of a first and second tube group (1, 2), including two tube holders (100, 200) each having a first (101, 201) and a second tube clamp (102, 202) and also a sealing blade (4),
wherein the tubes of the first tube group (1) can be inserted, specifically in a continuous manner, between the two tube holders (100, 200) in passage openings (11, 21) of the two first tube clamps (101, 201) and pinched therein,
wherein the tubes of the second tube group (2) can be inserted, specifically in a continuous manner, between the two tube holders (100, 200) in passage openings (12, 22) of the two second tube clamps (102, 202) and pinched therein,
wherein the sealing blade (4) can be moved between the first and the second tube holder (100, 200) and is configured in the process to sever the pinched tubes of the first and the second tube group (1, 2) in one movement in order to create cut ends (1', 2') and remaining ends (1", 2") in this way,
wherein one of the two tube holders (100, 200) can be displaced relative to the other tube holder (100, 200) in order to align the cut ends (1', 2') of the tubes with one another as a result, and
wherein the two tube holders (100, 200) can be displaced in a horizontal direction towards one another or away from one another, in order as a result to seal a respective cut end (1') of a tube of the first tube group (1) to a respective cut end (2') of a tube of the second tube group (2) to form a respective continuous tube at the same time, **characterized in that** the passage openings (11, 21) of the two first tube clamps (101, 201) are arranged on a first plane and the passage openings (12, 22) of the two second tube clamps (102, 202) are arranged on a second plane, wherein the first plane is arranged parallel to and spaced apart from the second plane.

2. Device according to Claim 1, **characterized in that** the two first tube clamps (101, 201) and the two second tube clamps (102, 202) have pinching zones (31, 32), wherein these pinching zones (31, 32) run along the passage openings (11, 21, 12, 22) and are configured to pinch the inserted tubes at an oblique angle to the first plane and/or to the second plane.

3. Device according to either of the preceding claims, **characterized in that** the sealing blade (4) can be clamped in a sealing blade holder (40) and a drive unit (401) is configured to move the sealing blade holder (40) in a vertical direction for the purpose of severing the tubes and in the process one of the two tube holders (100, 200) can be displaced vertically relative to the other tube holder (100, 200) to align the cut ends (1', 2') of the tubes.

4. Device according to Claim 3, **characterized in that** the movement of the sealing blade holder (40) in the vertical direction also makes it possible to move the two tube holders (100, 200) in the horizontal direction towards one another or away from one another or to move the two tube holders (100, 200) at the same time in one direction, and preferably wherein the device comprises a platform (400) which can be moved in the vertical direction and the sealing blade holder (40) is arranged on this platform (400).

5. Device according to either of Claims 3 and 4, **characterized in that** the sealing blade holder (40) comprises two oppositely situated clamping parts (43, 44), between which the sealing blade (4) can be clamped in, preferably one of the two clamping parts is arranged in a fixed manner and the other is arranged such that it can be moved relative to the fixed clamping part.

6. Device according to Claim 5, **characterized in that** provided on at least one of the two clamping parts (43, 44) is a pair of first slotted guide carriers (41), and the pair of slotted guide carriers (41) comprises preferably four first slotted guides (411) overall, wherein these slotted guides (411) are arranged identically among one another and mirror-symmetrically with respect to a plane of the sealing blade (4) when the sealing blade (4) is clamped in between the two clamping parts.

7. Device according to Claim 6, **characterized in that** sensing wheel pairs (53) are provided to sense the slotted guides (411) when the sealing blade holder (40) is moving in the vertical direction and, in the process, the two tube holders (100, 200) can be moved in the horizontal direction towards one another, or away from one another, or the two tube holders (100, 200) can be moved at the same time in one direction.

8. Device according to one of Claims 5 to 7, **characterized in that** a second slotted guide carrier (45) and a further sensing wheel (52) are provided, wherein the further sensing wheel (52) is configured to sense the second slotted guide carrier (45) when the sealing blade holder (40) is moving in the vertical direction, in order in the process to move the one clamping part (43) relative to the second, fixed clamping part (44), and the spacing between the two clamping parts (43, 44) can be varied in this way.

9. Device according to Claim 8, **characterized in that** the second slotted guide carrier (45) is sensed by the further sensing wheel (52), wherein three different positions are enabled, specifically a first contacting position (A) in which the spacing between the two clamping parts is selected such that the sealing blade (4) is clamped in and at the same time is electrically contacted, a second position (C) in which the spacing between the two clamping parts (43, 44) is selected such that the sealing blade can be ejected, and a receiving position (B) in which the spacing between the two clamping parts (43, 44) is selected such that the sealing blade can be pushed in between the two clamping parts (43, 44).

10. Device according to Claim 9, **characterized in that** arranged on at least one of the two clamping parts (43, 44) are thrust pieces, which make contact with the sealing blade (4) in the contacting position (A), wherein these thrust pieces are mounted resiliently on the at least one clamping part (43, 44).

11. Device according to one of Claims 5 to 10, **characterized in that** the device also comprises a transformer unit (90) having a primary winding and a secondary winding, wherein the secondary winding is formed by a single conductor and this conductor is connected to one of the two clamping parts (43, 44), preferably to the fixed clamping part (44), wherein the sealing blade (4) closes the secondary electrical circuit in the contacting position (A) and in this way is heated to the desired temperature.

12. Device according to one of the preceding claims, **characterized in that** the first and the second tube group (1, 2) each comprise the same number of individual tubes, preferably more than two, in particular six tubes, per tube group (1, 2).

13. Method for sealing a multiplicity of tubes of a first and a second tube group (1, 2) with a device according to one of the preceding Claims 1 to 12, wherein in one step the tubes of the first and the second tube group are inserted and pinched, specifically in a continuous manner, in parallel and one on top of another into oppositely situated first (100) and second tube holders (200), and a heatable sealing blade (4) is inserted into a sealing blade holder (4) and heated to a desired temperature,
the heated sealing blade (4) is moved between the first and the second tube holder (100, 200) and in the process the pinched tubes are severed at the same time into cut ends (1', 2') and remaining ends (1", 2"),
the cut ends (1', 2') of the tubes are melted by the heated sealing blade (4),
the cut ends (1') of the tubes of the first tube group (1) are aligned to the cut ends (2') of the tubes of the second tube group (2), wherein one of the two tube holders (100, 200) is displaced relative to the other tube holder (100, 200) with the result that the cut ends (1') of the tubes of the first tube group (1) are arranged symmetrically and oppositely situated to the cut ends (2') of the second tube group (2),
the two tube holders (100, 200) are pushed towards one another, in order as a result to seal a respective cut end (1') of the tubes of the first tube group (1) to a respective cut end (2') of the tubes of the second tube group (2) to form a respective continuous tube at the same time,
the sealing blade (4) is ejected from the sealing blade holder (40).

14. Method according to Claim 13, **characterized in that**, when inserting the sealing blade (4), the horizontal spacing between the first and the second tube holder (100, 200) is d1; when severing the pinched tubes, the spacing is increased to d4; when melting the cut ends, the spacing is d2, wherein the following applies: d1 < d2 < d4; when displacing one of the two tube holders (100, 200), the spacing is increased again to d4; during the sealing, the spacing is d3, wherein the following applies: d3 < d4; and, when ejecting the sealing blade from the cut ends (1', 2'), the spacing between the two tube holders (100, 200) is d5, wherein d5 constitutes the smallest of all five spacings (d1, d2, d3, d4).

## Revendications

1. Dispositif de soudage de tubes thermoplastiques d'un premier et d'un deuxième groupe de tubes (1, 2), comprenant deux porte-tubes (100, 200) dotés chacun d'un premier (101, 201) et d'un deuxième serre-tube (102, 202), ainsi qu'une lame de soudage (4),
dans lequel les tubes du premier groupe de tubes (1) sont insérés tout droit entre les deux porte-tubes (100, 200) à travers les ouvertures de passage (11, 21) des deux premiers serre-tubes (101, 201) et peuvent donc être pressés dans ceux-ci,
dans lequel les tubes du deuxième groupe de tubes (2) sont insérés tout droit entre les deux porte-tubes (100, 200) à travers les ouvertures de passage (12, 22) des deux deuxièmes serre-tubes (102, 202) et peuvent donc être pressés dans ceux-ci,
dans lequel la lame de soudage (4) peut être déplacée entre le premier et le deuxième porte-tubes (100, 200) et est disposée de façon à sectionner les tubes pressés du premier et du deuxième groupe de tubes (1, 2) afin d'obtenir de cette manière des bouts coupés (1', 2') et des bouts résiduels (1", 2"),
dans lequel un des deux porte-tubes (100, 200) peut être décalé relativement à l'autre porte-tubes (100, 200) afin d'aligner ainsi les bouts coupés (1', 2') des tubes, et dans lequel les deux porte-tubes (100, 200) peuvent être rapprochés l'un de l'autre ou éloignés l'un de l'autre dans une direction horizontale, afin de souder ainsi simultanément le bout coupé (1') d'un tube du premier groupe de tubes (1) avec un bout coupé (2') d'un tube du deuxième groupe de tubes (2) en un tube continu, **caractérisé en ce que** les ouvertures de passage (11, 21) des deux premiers serre-tubes (101, 201) sont disposées sur un premier plan et les ouvertures de passage (12, 22) des deux deuxièmes serre-tubes (102, 202) sont disposées sur un deuxième plan, dans lequel le premier plan est disposé parallèlement au deuxième plan et à distance de celui-ci.

2. Dispositif selon la revendication 1, **caractérisé en ce que** les deux premiers serre-tubes (101, 201) et les deux deuxièmes serre-tubes (102, 202) comportent des zones de pressage (31, 32), dans lequel ces zones de pressage (31, 32) longent les ouvertures de passage (11, 21, 12, 22) et sont disposées de façon à presser les tubes insérés dans un angle incliné par rapport au premier ou au deuxième plan.

3. Dispositif selon l'une des revendications précédentes, **caractérisé en ce que** la lame de soudage (4) peut être serrée dans un porte-lame de soudage (40) et une unité motrice (401) est disposée pour déplacer le porte-lame de soudage (40) afin de sectionner les tubes dans une direction verticale et ainsi un des deux porte-tubes (100, 200) peut être décalé verticalement relativement à l'autre porte-tube (100, 200) afin d'aligner les bouts coupés (1', 2') des tubes.

4. Dispositif selon la revendication 3, **caractérisé en ce qu'**en outre, par le mouvement du porte-lame de soudage (40) dans la direction verticale, les deux porte-tubes (100, 200) peuvent être rapprochés ou éloignés l'un de l'autre dans la direction horizontale ou les deux porte-tubes (100, 200) peuvent être déplacés simultanément dans une direction, et de préférence dans lequel le dispositif comporte une plateforme (400) mobile dans la direction verticale et le porte-lame de soudage (40) est disposé sur cette plateforme (400).

5. Dispositif selon l'une des revendications 3 à 4, **caractérisé en ce que** le porte-lame de soudage (40) comporte deux pièces de serrage (43, 44) se faisant face, entre lesquelles la lame de soudage (4) peut être serrée, de préférence une des deux pièces de serrage est disposée de manière fixe et l'autre est disposée de manière mobile relativement à la pièce de serrage fixe.

6. Dispositif selon la revendication 5, **caractérisé en ce que** sur au moins une des deux pièces de serrage (43, 44) est prévue une paire de premiers supports à glissière (41) et la paire de supports à glissière (41) comporte de préférence en tout quatre premières glissières (411), dans lequel ces glissières (411) sont identiques l'une à l'autre et sont disposées en symétrie de réflexion par rapport à un plan de la lame de soudage (4), lorsque la lame de soudage (4) est serrée entre les deux pièces de serrage.

7. Dispositif selon la revendication 6, **caractérisé en ce que** des paires de roues de balayage (53) sont prévues afin de balayer les glissières (411) lors du mouvement du porte-lame de soudage (40) dans la direction verticale et ainsi les deux porte-tubes (100, 200) peuvent être rapprochés ou éloignés l'un de l'autre dans la direction horizontale ou les deux porte-tubes (100, 200) peuvent être déplacés simultanément dans une direction.

8. Dispositif selon l'une des revendications 5 à 7, **caractérisé en ce qu'**un deuxième support à glissière (45) et une roue de balayage supplémentaire (52) sont prévus, dans lequel la roue de balayage supplémentaire (52) est disposée de façon à balayer le deuxième support à glissière (45) lors du mouvement du porte-lame de soudage (40) dans la direction verticale afin de déplacer ainsi l'une pièce de serrage (43) relativement à la deuxième pièce de serrage fixe (44) et de cette manière la distance entre les deux pièces de serrage (43, 44) est variable.

9. Dispositif selon la revendication 8, **caractérisé en ce que** le balayage du deuxième support à glissière (45) s'effectue par la roue de balayage supplémentaire (52), dans lequel trois positions différentes sont rendues possibles, à savoir une première position de mise en contact (A) pour laquelle la distance entre les deux pièces de serrage est sélectionnée de sorte que la lame de soudage (4) est serrée et simultanément en contact électrique, une deuxième position (C) pour laquelle la distance entre les deux pièces de serrage (43, 44) est sélectionnée de sorte que la lame de soudage peut être éjectée et une position d'accueil (B) pour laquelle la distance entre les deux pièces de serrage (43, 44) est sélectionnée de sorte que la lame de soudage peut être insérée entre les deux pièces de serrage (43, 44).

10. Dispositif selon la revendication 9, **caractérisé en ce que** des parties de pression, lesquelles font entrer la lame de soudage (4) en contact dans la position de mise en contact (A), sont disposées sur au moins une des deux pièces de serrage (43, 44), dans lequel ces parties de pression sont disposées de manière élastique sur l'au moins une pièce de serrage (43, 44).

11. Dispositif selon l'une des revendications 5 à 10, **caractérisé en ce que** le dispositif comporte en outre une unité de transformateur (90) dotée d'un enroulement primaire et d'un enroulement secondaire, dans lequel l'enroulement secondaire est formé par un unique conducteur et ce conducteur est relié avec une des deux pièces de serrage (43, 44), de préférence avec la pièce de serrage fixe (44), dans lequel la lame de soudage (4) ferme la boucle de courant secondaire dans la position de mise en contact (A) et est de cette manière chauffée à la température souhaitée.

12. Dispositif selon l'une des revendications précédentes, **caractérisé en ce que** le premier et le deuxième groupe de tubes (1, 2) comportent chacun le même nombre de tubes individuels, de préférence plus de deux, en particulier six tubes, par groupe de tubes (1, 2).

13. Procédé de soudage d'une pluralité de tubes d'un premier et d'un deuxième groupe de tubes (1, 2) avec un dispositif selon l'une des revendications précédentes 1 à 12,
dans lequel dans une étape les tubes d'un premier et d'un deuxième groupe de tubes sont insérés tout droit parallèlement et l'un sur l'autre à travers un premier (100) et un deuxième porte-tubes (200) se faisant face et pressés et une lame de soudage chauffable (4) est insérée dans un porte-lame de soudage (4) et chauffée jusqu'à une température souhaitée, la lame de soudage (4) chauffée est déplacée entre le premier et le deuxième porte-tubes (100, 200) et ainsi les tubes pressés sont simultanément sectionnés en bouts coupés (1', 2') et en bouts résiduels (1", 2"),
les bouts coupés (1', 2') des tubes sont fondus par la lame de soudage (4) chauffée, les bouts coupés (1') des tubes du premier groupe de tubes (1) sont alignés sur les bouts coupés (2') des tubes du premier groupe de tubes (2), dans lequel un des deux porte-tubes (100, 200) est décalé relativement à l'autre porte-tubes (100, 200), de sorte que les bouts coupés (1') des tubes du premier groupe de tubes (1) sont disposés en opposition symétrique par rapport aux bouts coupés (2') des tubes du premier groupe de tubes (2),
les deux porte-tubes (100, 200) sont rapprochés l'un de l'autre, afin que le bout coupé (1') d'un tube du premier groupe de tubes (1) soit ainsi soudé simultanément avec un bout coupé (2') d'un tube du deuxième groupe de tubes (2) en un tube continu,
la lame de soudage (4) est éjectée du porte-lame de soudage (40).

14. Procédé selon la revendication 13, **caractérisé en ce que** lors de l'insertion de la lame de soudage (4), la distance horizontale entre le premier et le deuxième porte-tubes (100, 200) est d1, lors de la section des tubes pressés la distance est augmentée jusqu'à d4, lors de la fusion des bouts coupés la distance vaut d2, dans lequel : d1<d2<d4, lors du décalage d'un des deux porte-tubes (100, 200) la distance est de nouveau augmentée jusqu'à d4, lors du soudage la distance est d3, dans lequel d3<d4 et lors de l'éjection de la lame de soudage des bouts coupés (1', 2') la distance entre les deux porte-tubes (100, 200) vaut d5, dans lequel d5 constitue la plus petite des cinq distances (d1, d2, d3, d4).
